# EUROPEAN PATENT SPECIFICATION

(11) **EP 2 333 081 B1**
(45) Date of publication and mention of the grant of the patent: **09.03.2016**
(21) Application number: 10015540.7
(22) Date of filing: 13.12.2007
(51) Int. Cl.: C12N 15/82, A01H 1/00

(54) **Tabacco plants having reduced nicotine demethylase activity**
Tabakpflanzen mit verminderter Nikotindemethylseaktivität
Plantes de tabac bénéficiant d'une activité de déméthylase de nicotine réduite

(30) Priority: 15.12.2006 US 611782
(43) Date of publication of application: 15.06.2011
(62) Divisional of application: 07865628.7
(73) Proprietor: U.S. Smokeless Tobacco Company LLC, Richmond, VA 23230 (US)
(72) Inventor: Xu, Dongmei, Lexington, KY 40515 (US); Nielsen, Mark T., Madison, TN 37115 (US); Shen, Yanxin, Glen Allen, VA 23059 (US)
(74) Representative: Wichmann, Hendrik

(56) References cited:
- WO-A2-2004/035745
- WO-A2-2005/111217
- WO-A2-2008/076802
- US-A1- 2005 223 442
- US-A1- 2006 041 949
- US-A1- 2007 199 097

## Description

### BACKGROUND OF THE INVENTION

### 1. Technical Field

The present invention is generally directed to mutant tobacco plants having reduced nicotine demethylase activity, a method of making such a mutant tobacco plant, and progenies of such plants, as defined in the claims.

### 2. Background Information

Tobacco plants are known to N-demethylate nicotine to form nornicotine, a secondary alkaloid known to be a precursor for the microbial-mediated formation of N-Nitrosonomicotine (hereinafter, "NNN") in cured leaves. The N-demethylation reaction is catalyzed by the enzyme nicotine demethylase (NDM). Current methods to reduce the conversion of the substrate nicotine to the product nornicotine in tobacco have utilized screening to eliminate converter plants from foundation seed lots that are used for commercial seed production. Seed produced directly from screened seed, however, still contains converters. WO 2005/111217 and US 2006/004194 describe plants having an altered expression level of nicotine demethylase and methods to obtain such plants.

### SUMMARY OF THE INVENTION

Provided herein are methods related to the production of tobacco plants, having a mutation in a nicotine demethylase gene.

Provided herein are tobacco plants having a mutation in a nicotine demethylase gene. A plant having a mutation in a nicotine demethylase gene can have a non-converter phenotype, and the progeny of such a plant can have a reversion rate that is reduced at least 2X *(e.g.,* 10X to 1000X or 2X to 100X) compared to the reversion rate of the corresponding tobacco plant comprising a wild type nicotine demethylase gene. A tobacco plant can be a Burley type, a dark type, a flue-cured type, or an Oriental type tobacco, or a *Nicotiana tabacum.* A plant can be essentially derived from BU 64, CC 101, CC 200, CC 27, CC 301, CC 400, CC 500, CC 600, CC 700, CC 800, CC 900, Coker 176, Coker 319, Coker 371 Gold, Coker 48, CU 263, DF911, Galpao tobacco, GL 26H, GL 350, GL 600, GL 737, GL 939, GL 973, HB 04P, K 149, K 326, K 346, K 358, K394, K 399, K 730, KDH 959, KT 200, KT204LC, KY 10, KY 14, KY 160, KY 17, KY 171, KY 907, KY907LC, KTY14 x L8 LC, Little Crittenden, McNair 373, McNair 944, msKY 14xL8, Narrow Leaf Madole, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, VA 309, or VA359.

Also provided are tobacco plants having a mutant allele at a nicotine demethylase locus. In certain embodiments, a mutant allele at a nicotine demethylase locus encodes an amino acid sequence selected from the group consisting of: SEQ ID NO:2, wherein the proline at amino acid 107 is replaced with a with an amino acid selected from the group consisting of alanine, arginine, asparagine, aspartic acid, cysteine, glutamic acid, glutamine, histidine, isoleucine, leucine, lysine, methionine, phenylalanine, serine, threonine, tryptophan, tyrosine, and valine; SEQ ID NO:2, wherein an amino acid P107 is deleted. In one particular embodiment, a mutant allele encodes an amino acid sequence comprising the sequence set forth in SEQ ID NO:2, wherein the proline at amino acid 107 is replaced with a leucine.

In certain other embodiments, the invention is directed to methods of making a tobacco plant, as defined in the clams. In particular embodiments, a method of making a tobacco plant comprises inducing mutagenesis in cells of a *Nicotiana* species, obtaining one or more plants from said cells, and identifying at least one of such plants that contains a nicotine demethylase gene having at least one mutation. The method may further comprise crossing a plant containing said at least one mutation in a nicotine demethylase gene with a second *Nicotiana* plant; and selecting progeny of the cross that have the nicotine demethylase gene mutation. The mutation comprises a nicotine demethylase gene encoding the amino acid sequence set forth in SEQ ID NO:2, wherein the proline at amino acid 107 is replaced with a with an amino acid selected from the group consisting of alanine, arginine, asparagine, aspartic acid, cysteine, glutamic acid, glutamine, histidine, isoleucine, leucine, lysine, methionine, phenylalanine, serine, threonine, tryptophan, tyrosine, and valine; a nicotine demethylase gene encoding the amino acid sequence set forth in SEQ ID NO:2, wherein an amino acid P107 is deleted. In particular embodiments, inducing mutagenesis in cells of a *Nicotiana* species are in a seed.

The second tobacco plant may exhibit a phenotypic trait such as disease resistance; high yield; high grade index; curability; curing quality; mechanical harvestability; holding ability; leaf quality; height, plant maturation (e.g., early maturing, early to medium maturing, medium maturing, medium to late maturing, or late maturing); stalk size (e.g., a small, medium, or a large stalk); or leaf number per plant (e.g., a small (e.g., 5-10 leaves), medium (e.g., 11-15 leaves), or large (e.g., 16-21) number of leaves). The method may further include self-pollinating or pollinating a male sterile pollen acceptor with a pollen donor capable of being used in production of a hybrid or a male sterile hybrid. Either the male sterile pollen acceptor plant or the pollen donor plant has a mutant allele at a nicotine demethylase locus. In some embodiments, both alleles at the nicotine demethylase locus are mutant alleles.

Also provided herein is cured tobacco material. The cured tobacco is made from plants having a mutation in a nicotine demethylase gene. The tobacco plant having a mutation in a nicotine demethylase gene has a non-converter phenotype.Cured tobacco material is made by a curing process selected from the group consisting of flue curing, air curing, fire curing and sun curing.

Also provided herein are tobacco products. A tobacco product may comprise cured tobacco material obtained from plants having a mutant allele at a nicotine demethylase locus. A tobacco product may be a cigarette product, a cigar product, a pipe tobacco product, a smokeless tobacco product, a film, a tab, a gel, a shaped part, a rod, or a foam.

Provided herein are M₁ tobacco plants and progeny of M₁ tobacco plants. An M₁ tobacco plant can be heterozygous for a mutant allele at a nicotine demethylase locus and produce progeny, wherein at least a portion of first generation self-pollinated progeny of said plant exhibit a non-converter phenotype. Progeny of said M₁ tobacco plant can revert to a converter phenotype at a rate that is statistically significantly less than the reversion rate of the progeny of the corresponding tobacco plant that comprises a wild type allele at said nicotine demethylase locus. An M₁ tobacco plant can exhibit a non-converter phenotype and produce progeny that can revert to a converter phenotype at a rate that is statistically significantly less than the reversion rate of the progeny of a corresponding tobacco plant that comprises a wild type allele at said nicotine demethylase locus. A plant or progeny may be essentially derived from BU 64, CC 101, CC 200, CC 27, CC 301, CC 400, CC 500, CC 600, CC 700, CC 800, CC 900, Coker 176, Coker 319, Coker 371 Gold, Coker 48, CU 263, DF911, Galpao tobacco, GL 26H, GL 350, GL 600, GL 737, GL 939, GL 973, HB 04P, K 149, K 326, K 346, K 358, K394, K 399, K 730, KDH 959, KT 200, KT204LC, KY 10, KY 14, KY 160, KY 17, KY 171, KY 907, KY907LC, KTY14 x L8 LC, Little Crittenden, McNair 373, McNair 944, msKY 14xL8, Narrow Leaf Madole, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TN 86, TN86LC, TN 90, TN 97, TN97LC, TN D94, TN D950, TR (Tom Rosson) Madole, VA 309, or VA359.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the detailed description set forth below. Other features, objects, and advantages of the invention will be apparent from the description and drawings, and from the claims.

### DESCRIPTION OF THE DRAWINGS

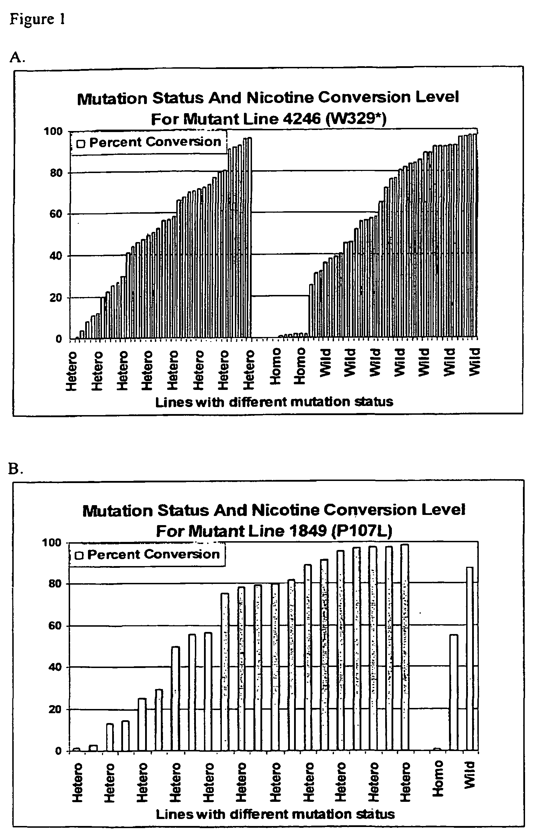

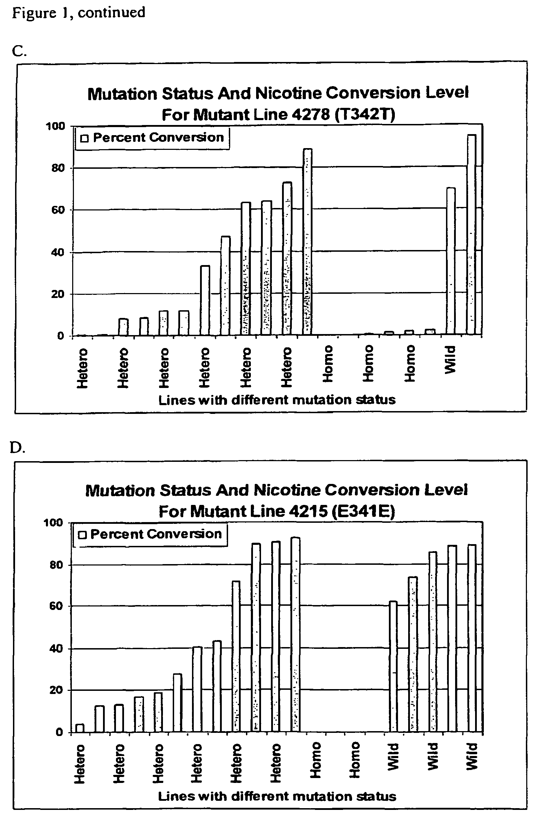

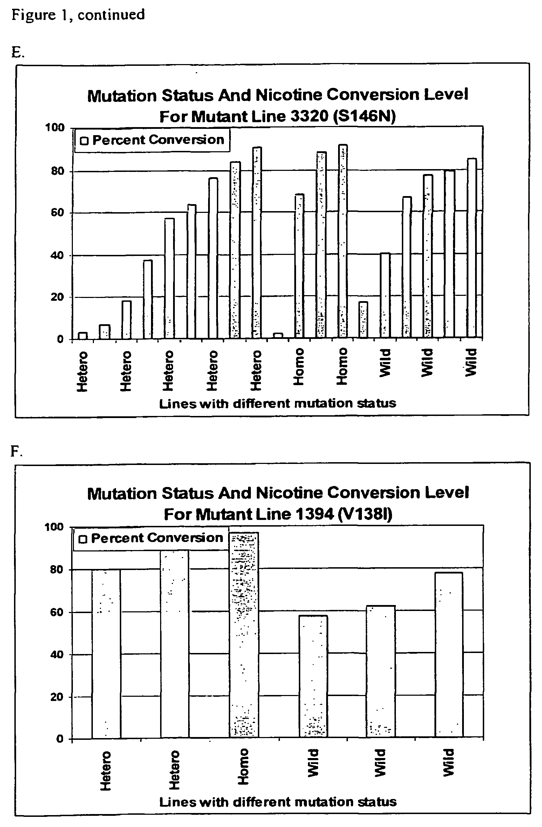

Figure 1 depicts percent conversion of nicotine to nornicotine as measured by gas chromatography in ethylene-treated leaves of TN90 tobacco lines relative to mutation status. A: Line 4246, B: Line 1849, C: Line 4278, D: Line 4215, E: Line 3320, and F: Line 1394. "Hetero" indicates the plant is heterozygous for a mutant nicotine demethylase allele. "Homo" indicates the plant is homozygous for a mutant nicotine demethylase allele. "Wild" indicates the plant is homozygous for wild-type nicotine demethylase.

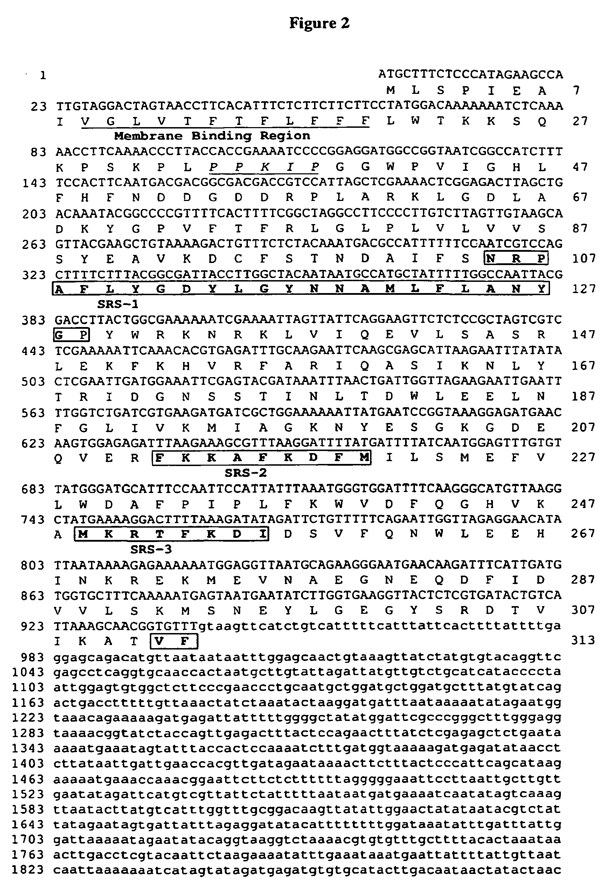

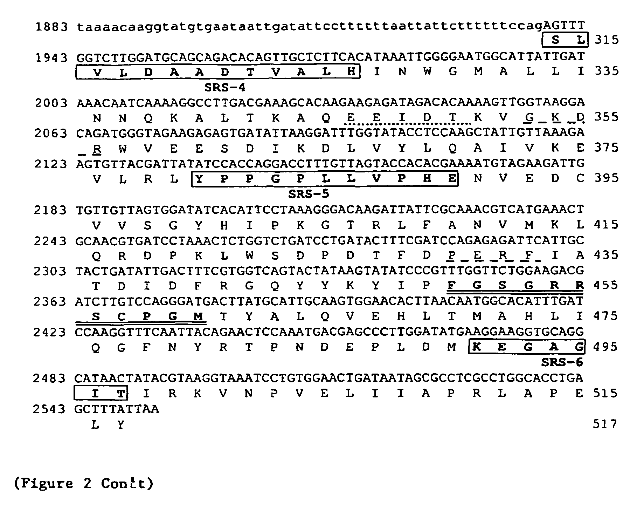

Figure 2 shows a nicotine demethylase nucleic acid sequence (SEQ ID NO:1) and amino acid sequence (SEQ ID NO:2). Numbers corresponding to the nucleotide sequence are on the left side and numbers corresponding to the amino acid sequence are on the right side. The Web Signal Scan program and sequence alignment tools were used to identify the following: substrate recognition sites (boxed), N-terminal hydrophobic transmembrane domain (underlined), proline-rich region (underlined and in italics), threonine-containing oxygen-binding pocket (dotted underlined), K-helix and PERF consensus (dashed underlined), and cysteine-containing heme-binding region (double underlined and in bold).

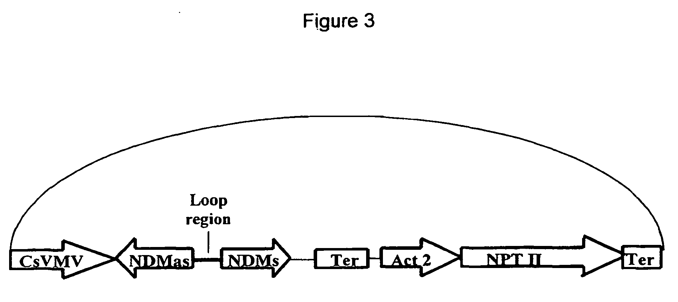

Figure 3 shows a schematic of a nicotine demethylase RNAi construct. CsVMV-Cassava vein mosaic virus promoter; NDMas-antisense nicotine demethylase sequence; NDMs-sense nicotine demethylase sequence; Ter-*Nos* terminator; Act2-*Arabidopsis thaliana* Actin 2 promoter; NPTII-neomycin phosphotransferase II gene.

### DETAILED DESCRIPTION

The present invention is directed to methods related to tobacco plants having reduced nicotine demethylase activity, as defined in the claims. For example, this document provides tobacco plants having a mutation in a nicotine demethylase gene, as defined in the claims. Such tobacco plants comprising a mutant nicotine demethylase sequence in its genome typically have a reduced nornicotine content. Such plants are useful in tobacco breeding programs, in making cured tobacco and in making various tobacco products and/or tobacco derived products.

### Mutations in a nicotine demethylase gene

Tobacco plants described herein are typically generated by inducing mutagenesis in cells of a *Nicotiana* species. The term "mutagenesis" refers to the use of a mutagenic agent to induce genetic mutations within a population of individuals. A population to be mutagenized can comprise plants, parts of plants, or seeds. For mutagenized populations the dosage of the mutagenic chemical or radiation is determined experimentally for each type of plant tissue such that a mutation frequency is obtained that is below a threshold level characterized by lethality or reproductive sterility. The number of M₁ generation seed or the size of M₁ plant populations resulting from the mutagenic treatments are estimated based upon the expected frequency of mutations.

The mutagenized population, or a subsequent generation of that population, is then screened for a desired trait(s) (*e.g*., a non-converter phenotype) that results from the mutation(s). Alternatively, the mutagenized population, or a subsequent generation of that population, is screened for a mutation in a gene of interest, e.g., a nicotine demethylase gene. For example, the progeny M₂ generation of M₁ plants may be evaluated for the presence of a mutation in a nicotine demethylase gene. A "population" is any group of individuals that share a common gene pool. As used herein, "M₀" refers to plant cells(and plants grown therefrom) exposed to a mutagenic agent, while "M₁" refers to seeds produced by self-pollinated M₀ plants, and plants grown from such seeds. "M₂" is the progeny (seeds and plants) of self-pollinated M₁ plants, "M₃" is the progeny of self-pollinated M₂ plants, and "M₄" is the progeny of self-pollinated M₃ plants. "M₅" is the progeny of self-pollinated M₄ plants. "M₆", "M₇", etc. are each the progeny of self-pollinated plants of the previous generation. The term "selfed" as used herein means self-pollinated.

Suitable mutagenic agents include, for example, chemical mutagens and ionizing radiation. Chemical mutagens suitable for inducing mutations include nitrous acid, sodium azide, acridine orange, ethidium bromide and ethyl methane sulfonate. Ionizing radiation suitable for inducing mutations includes X-rays, gamma rays, fast neutron irradiation and UV radiation. Other methods include the use of transposons (Fedoroff et al., 1984; U.S. Pat. No. 4,732,856 and U.S. Pat. No. 5,013,658), as well as T-DNA insertion methodologies (Hoekema et al., 1983; U.S. Pat. No. 5,149,645). The types of mutations that may be induced in a tobacco gene include, for example, point mutations, deletions, insertions, duplications, and inversions.

In some embodiments, mutagenesis is induced by growing plant cells in tissue culture, which results in the production of somaclonal variants. Alternatively, application of standard protoplast culture methodologies developed for production of hybrid plants using protoplast fusion is also useful for generating plants having variant gene expression (e.g., variant nicotine demethylase gene expression). Accordingly, protoplasts are generated from a first and a second tobacco plant having variant gene expression. Calli are cultured from successful protoplast fusions and plants are then regenerated. Resulting progeny hybrid plants are identified and selected for variant gene expression according to methods described herein and may be used in a breeding protocols described herein.

The term "nicotine demethylase gene" as used herein refers to a genomic nucleic acid sequence encoding a nicotine demethylase polypeptide. A nicotine demethylase gene includes coding sequences at a nicotine demethylase locus, as well as noncoding sequences such as regulatory regions, introns, and other untranslated sequences. A wild-type nicotine demethylase gene can comprise the nucleic acid sequence set forth in SEQ ID NO:1. The term "nicotine demethylase polypeptide" as used herein refers to a cytochrome P450 CYP82E4 polypeptide having nicotine demethylase activity. "Nicotine demethylase activity" is the ability to N'-demethylate nicotine to produce nornicotine. A wild-type nicotine demethylase polypeptide can comprise the amino acid sequence set forth in SEQ ID NO:2.

As provided herein (e.g., in Figure 2 and Example 5), a nicotine demethylase polypeptide can contain regions having homology with conserved domains in other cytochrome P450 polypeptides. For example, a polypeptide having the sequence set forth in SEQ ID NO:2 contains six substrate recognition sites (SRS), an N-terminal hydrophobic transmembrane domain, a proline-rich region, a threonine-containing oxygen-binding pocket, a K-helix consensus, a PERF consensus, and a cysteine-containing heme-binding region, as identified by the TFSEARCH and Web Signal Scan programs. See Figure 2. The K-helix and PERF consensus sequences are thought to stabilize the core structure of cytochrome P450 polypeptides. The heme-binding region contains a cysteine that is absolutely conserved in electron donor-dependent cytochrome P450 polypeptides. The proline-rich region is thought to form a hinge between the transmembrane region and the globular part of the polypeptide. See, e.g., Werck-Reichhart and Feyereisen (2000) Genome Biology 1:3003.

Preferably, a mutation in a nicotine demethylase gene results in reduced or even complete elimination of nicotine demethylase activity in a plant comprising the mutation. Suitable types of mutations in a nicotine demethylase gene include, without limitation, insertions of nucleotides, deletions of nucleotides, or transitions or transversions in the wild-type nicotine demethylase gene sequence. Mutations in the coding sequence can result in insertions of one or more amino acids, deletions of one or more amino acids, and/or non-conservative amino acid substitutions in the corresponding gene product. In some cases, the sequence of a nicotine demethylase gene comprises more than one mutation or more than one type of mutation.

Insertion or deletion of amino acids in a coding sequence can, for example, disrupt the conformation of a substrate binding pocket of the resulting gene product. Amino acid insertions or deletions can also disrupt catalytic sites important for gene product activity (e.g., a heme-binding site). It is known in the art that the insertion or deletion of a larger number of contiguous amino acids is more likely to render the gene product non-functional, compared to a smaller number of inserted or deleted amino acids. An example of such a mutation is a mutation in a nicotine demethylase gene encoding the amino acid sequence set forth in SEQ ID NO:2, which mutation results in the tryptophan at amino acid 329 being replaced with a stop codon.

Non-conservative amino acid substitutions can replace an amino acid of one class with an amino acid of a different class. Non-conservative substitutions can make a substantial change in the charge or hydrophobicity of the gene product. Non-conservative amino acid substitutions can also make a substantial change in the bulk of the residue side chain, *e.g*., substituting an alanine residue for a isoleucine residue. Examples of non-conservative substitutions include a basic amino acid for a non-polar amino acid, or a polar amino acid for an acidic amino acid. An example of such mutations is a mutation in a nicotine demethylase gene encoding the amino acid sequence set forth in SEQ ID NO:2, which mutation results in the proline at amino acid 107 being replaced by a leucine.

In some embodiments, a mutation in a nicotine demethylase gene results in no amino acid changes (*e.g*., a silent mutation). Silent mutations are mutations in a nucleotide sequence that do not affect the amino acid sequence of the encoded polypeptide. Silent mutations effective for reducing nicotine demethylase activity include mutations in the nicotine demethylase gene of SEQ ID NO: 1, in which the guanine at nucleic acid +2021 is replaced with an adenine, or the guanine at nucleic acid +2291 is replaced with an adenine. Other mutations that result in no amino acid changes can be in a 5' noncoding region (*e.g*., a promoter or a 5' untranslated region), an intron, or a 3' noncoding region. Such mutations, although not affecting the amino acid sequence of the encoded nicotine demethylase, may alter transcriptional levels (*e.g*., increasing or decreasing transcription), decrease translational levels, alter secondary structure of DNA or mRNA, alter binding sites for transcriptional or translational machinery, or decrease tRNA binding efficiency. Suitable mutations that reduce or eliminate nicotine demethylase activity include mutations that insert a splice donor in the intron of the nicotine demethylase gene, insert a splice acceptor in the intron, or delete a splice site of the intron.

In certain embodiments, a mutation in a nicotine demethylase gene effective for reducing nicotine demethylase activity is determined by identifying a plant having a mutation in a nicotine demethylase gene and measuring nicotine demethylase enzyme activity. In other embodiments, a mutation in a nicotine demethylase gene that is suitable for reducing nicotine demethylase activity is predicted based on the effect of mutations described herein, e.g., those mutations contained in TN90 lines 4246, 1849, 4278, and 4215 as set forth in Table 1 and Table 3. For example, a mutation in a nicotine demethylase gene encoding the amino acid sequence set forth in SEQ ID NO:2 can include a mutation that replaces any of amino acids 1-328 with a stop codon.

In another embodiment, a mutation in a nicotine demethylase gene that is effective for reducing nicotine demethylase activity is identified based on the function of related sequences, *e.g*., SEQ ID NO:3 and SEQ ID NO:4. For example, a nicotine demethylase gene can be mutated such that it encodes a nicotine demethylase polypeptide having a combination of mutations in SEQ ID NO:2, such as the combination of I163M, K309E, G353C, and S452P, or the combination of Q416L and S423P.

In certain other embodiments, a mutation in a nicotine demethylase gene that is effective for reducing nicotine demethylase activity is identified based on a molecular model or sequence analysis of the structure of a nicotine demethylase polypeptide. Such a molecular model or sequence analysis can be used to identify which amino acids, when mutated, will change the structure or function of the polypeptide. For example, a molecular model can be used to identify which amino acids in a substrate binding pocket can be deleted or substituted with a nonconservative amino acid to reduce the level of conversion of nicotine to nornicotine. In another example, sequence analysis can determine which amino acids can be replaced with a stop codon to disrupt a conserved domain. For example, a mutation in a nicotine demethylase gene encoding the amino acid sequence set forth in SEQ ID NO:2 can include a mutation that replaces any of amino acids 330-457 with a stop codon, thereby disrupting the heme-binding site of nicotine demethylase.

### Tobacco Plants Having Mutant Nicotine Demethylase Alleles

One or more M₁ tobacco plants are obtained from cells of mutagenized individuals and at least one of the plants is identified as containing a mutation in a nicotine demethylase gene. An M₁ tobacco plant may be heterozygous for a mutant allele at a nicotine demethylase locus and, due to the presence of the wild-type allele, exhibit a converter phenotype, i.e., be capable of converting nicotine to nornicotine. In such cases, at least a portion of first generation self-pollinated progeny of such a plant exhibit a non-converter phenotype. Alternatively, an M₁ tobacco plant may have a mutant allele at a nicotine demethylase locus and exhibit a non-converter phenotype. Such plants may be heterozygous and exhibit a non-converter phenotype due to phenomena such a dominant negative suppression, despite the presence of the wild-type allele, or may be homozygous due to independently induced mutations in both alleles at the nicotine demethylase locus. Subsequent progeny of both types of M₁ plants, however, revert to a converter phenotype at a rate that is statistically significantly less than the reversion rate of the progeny of a corresponding tobacco plant that is wild type at the nicotine demethylase locus, as discussed below.

M₁ tobacco plants carrying mutant nicotine demethylase alleles can be from *Nicotiana* species such as *Nicotiana tabacum, Nicotiana otophora, Nicotiana thrysiflora, Nicotiana tomentosa, Nicotiana tomentosiformis, Nicotiana africana, Nicotiana amplexicaulis*, *Nicotiana arentsii, Nicotiana benthamiana, Nicotiana bigelovii, Nicotiana corymbosa*, *Nicotiana debneyi, Nicotiana excelsior, Nicotiana exigua, Nicotiana glutinosa, Nicotiana goodspeedii, Nicotiana gossei, Nicotiana hesperis, Nicotiana ingulba, Nicotiana knightiana, Nicotiana maritima, Nicotiana megalosiphon, Nicotiana miersii, Nicotiana nesophila, Nicotiana noctiflora, Nicotiana nudicaulis, Nicotiana otophora, Nicotiana palmeri, Nicotiana paniculata, Nicotiana petunioides, Nicotiana plumbaginifolia, Nicotiana repanda, Nicotiana rosulata, Nicotiana rotundifolia, Nicotiana rustica, Nicotiana setchelli, Nicotiana stocktonii, Nicotiana eastii, Nicotiana suaveolens* or *Nicotiana trigonophylla.*

Particularly useful *Nicotiana tabacum* varieties include Burley type, dark type, flue-cured type, and Oriental type tobaccos, such as tobacco varieties BU 64, CC 101, CC 200, CC 27, CC 301, CC 400, CC 500, CC 600, CC 700, CC 800, CC 900, Coker 176, Coker 319, Coker 371 Gold, Coker 48, CU 263, DF911, Galpao tobacco, GL 26H, GL 350, GL 600, GL 737, GL 939, GL 973, HB 04P, K 149, K 326, K 346, K 358, K394, K 399, K 730, KDH 959, KT 200, KT204LC, KY 10, KY 14, KY 160, KY 17, KY 171, KY 907, KY907LC, KTY14 x L8 LC, Little Crittenden, McNair 373, McNair 944, msKY 14xL8, Narrow Leaf Madole, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, VA 309, or VA359.

A tobacco plant carrying a mutant nicotine demethylase allele can be used in a plant breeding program to create novel and useful lines, varieties and hybrids. Thus, in some embodiments, an M₁, M₂, M₃, or later generation tobacco plant containing at least one mutation in a nicotine demethylase gene is crossed with a second *Nicotiana* plant, and progeny of the cross are identified in which the nicotine demethylase gene mutation(s) is present. It will be appreciated that the second *Nicotiana* plant can be one of the species and varieties described herein. It will also be appreciated that the second *Nicotiana* plant can contain the same nicotine demethylase mutation as the plant to which it is crossed, a different nicotine demethylase mutation, or be wild-type at the nicotine demethylase locus.

Breeding is carried out via known procedures. DNA fingerprinting, SNP or similar technologies may be used in a marker-assisted selection (MAS) breeding program to transfer or breed mutant alleles of a nicotine demethylase gene into other tobaccos, as described herein. For example, a breeder can create segregating populations from hybridizations of a genotype containing a mutant allele with an agronomically desirable genotype. Plants in the F₂ or backcross generations can be screened using a marker - developed from a nicotine demethylase sequence or a fragment thereof, using one of the techniques listed herein. Plants identified as possessing the mutant allele can be backcrossed or self-pollinated to create a second population to be screened. Depending on the expected inheritance pattern or the MAS technology used, it may be necessary to self-pollinate the selected plants before each cycle of backcrossing to aid identification of the desired individual plants. Backcrossing or other breeding procedure can be repeated until the desired phenotype of the recurrent parent is recovered.

Nicotiana species which exhibit breeding compatibility with *Nicotiana tabacum* include *Nicotiana amplexicaulis,* PI 271989; *Nicotiana benthamiana* PI 555478; *Nicotiana bigelovii* PI 555485; *Nicotiana debneyi; Nicotiana excelsior* PI 224063; *Nicotiana glutinosa* PI 555507; *Nicotiana goodspeedii* PI 241012; *Nicotiana gossei* PI 230953; *Nicotiana hesperis* PI 271991; *Nicotiana knightiana* PI 555527; *Nicotiana maritima* PI 555535; *Nicotiana megalosiphon* PI 555536; *Nicotiana nudicaulis* PI 555540; *Nicotiana paniculata* PI 555545; *Nicotiana plumbaginifolia* PI 555548; *Nicotiana repanda* PI 555552; *Nicotiana rustica; Nicotiana suaveolens* PI 230960; *Nicotiana sylvestris* PI 555569; *Nicotiana tomentosa* PI 266379; *Nicotiana tomentosiformis*; and *Nicotiana trigonophylla* PI 555572. See also, Compendium of Tobacco Diseases published by American Phytopathology Society, or The Genus Nicotiana Illustrated, published by Japan Tobacco Inc.

Successful crosses yield F₁ plants that are fertile and that can be backcrossed with one of the parents if desired. In some embodiments, a plant population in the F₂ generation is screened for variant nicotine demethylase gene expression, e.g., a plant is identified that fails to express nicotine demethylase due to the absence of a nicotine demethylase gene according to standard methods, for example, by using a PCR method with primers based upon the nucleotide sequence information for nicotine demethylase described herein. Selected plants are then crossed with one of the parents and the first backcross (BC₁) generation plants are self-pollinated to produce a BC₁F₂ population that is again screened for variant nicotine demethylase gene expression (e.g., the null version of the nicotine demethylase gene). The process of backcrossing, self-pollination, and screening is repeated, for example, at least 4 times until the final screening produces a plant that is fertile and reasonably similar to the recurrent parent. This plant, if desired, is self-pollinated and the progeny are subsequently screened again to confirm that the plant exhibits variant nicotine demethylase gene expression (e.g., a plant that displays the null condition for nicotine demethylase) or variant expression of NDM nucleic acid sequence, or a fragment thereof. Cytogenetic analyses of the selected plants are optionally performed to confirm the chromosome complement and chromosome pairing relationships. Breeder's seed of the selected plant is produced using standard methods including, for example, field testing, confirmation of the null condition for nicotine demethylase, chemical analyses of cured leaf to determine the level of alkaloids and/or chemical analyses of cured leaf to determine the ratio of nornicotine to nicotine+nornicotine.

In situations where the original F₁ hybrid resulting from the cross between a first, mutant tobacco parent (e.g., TN 90) and a second, wild-type tobacco parent (e.g., *N. rustica*), is hybridized or backcrossed to the mutant tobacco parent, the progeny of the backcross can be self-pollinated to create a BC₁F₂ generation that is screened for the mutant nicotine demethylase allele.

The result of a plant breeding program using the mutant tobacco plants described herein are novel and useful lines, hybrids and varieties. As used herein, the term "variety" refers to a population of plants that share constant characteristics which separate them from other plants of the same species. A variety is often, although not always, sold commercially. While possessing one or more distinctive traits, a variety is further characterized by a very small overall variation between individuals within that variety. A "pure line" variety may be created by several generations of self-pollination and selection, or vegetative propagation from a single parent using tissue or cell culture techniques. A variety can be essentially derived from another line or variety. As defined by the International Convention for the Protection of New Varieties of Plants (December 2, 1961, as revised at Geneva on November 10, 1972, on October 23, 1978, and on March 19, 1991), a variety is "essentially derived" from an initial variety if: a) it is predominantly derived from the initial variety, or from a variety that is predominantly derived from the initial variety, while retaining the expression of the essential characteristics that result from the genotype or combination of genotypes of the initial variety; b) it is clearly distinguishable from the initial variety; and c) except for the differences which result from the act of derivation, it conforms to the initial variety in the expression of the essential characteristics that result from the genotype or combination of genotypes of the initial variety. Essentially derived varieties can be obtained, for example, by the selection of a natural or induced mutant, a somaclonal variant, a variant individual from plants of the initial variety, backcrossing, or transformation. A "line" as distinguished from a variety most often denotes a group of plants used non-commercially, for example in plant research. A line typically displays little overall variation between individuals for one or more traits of interest, although there may be some variation between individuals for other traits.

Hybrid tobacco varieties can be produced by preventing self-pollination of female parent plants (i.e., seed parents) of a first variety, permitting pollen from male parent plants of a second variety to fertilize the female parent plants, and allowing F₁ hybrid seeds to form on the female plants. Self-pollination of female plants can be prevented by emasculating the flowers at an early stage of flower development. Alternatively, pollen formation can be prevented on the female parent plants using a form of male sterility. For example, male sterility can be produced by cytoplasmic male sterility (CMS), nuclear male sterility, genetic male sterility, molecular male sterility wherein a transgene inhibits microsporogenesis and/or pollen formation, or self-incompatibility. Female parent plants containing CMS are particularly useful. In embodiments in which the female parent plants are CMS, the male parent plants typically contain a fertility restorer gene to ensure that the F₁ hybrids are fertile. In other embodiments in which the female parents are CMS, male parents can be used that do not contain a fertility restorer. F₁ hybrids produced from such parents are male sterile. Male sterile hybrid seed can be interplanted with male fertile seed to provide pollen for seed-set on the resulting male sterile plants.

Varieties and lines described herein can be used to form single-cross tobacco F₁ hybrids. In such embodiments, the plants of the parent varieties can be grown as substantially homogeneous adjoining populations to facilitate natural cross-pollination from the male parent plants to the female parent plants. The F₁ seed formed on the female parent plants is selectively harvested by conventional means. One also can grow the two parent plant varieties in bulk and harvest a blend of F₁ hybrid seed formed on the female parent and seed formed upon the male parent as the result of self-pollination. Alternatively, three-way crosses can be carried out wherein a single-cross F₁ hybrid is used as a female parent and is crossed with a different male parent. As another alternative, double-cross hybrids can be created wherein the F₁ progeny of two different single-crosses are themselves crossed. Self-incompatibility can be used to particular advantage to prevent self-pollination of female parents when forming a double-cross hybrid.

As used herein, a tobacco plant having a converter phenotype is a tobacco plant having a percent nicotine demethylation of at least 5% *(e.g.,* 5.0%, 5.1%, 5.5%, 6%, 8%, 15%, 30%, 50%, 70%, 90%, 95%, 98%, or 99%) as measured in an ethylene-treated middle position leaf harvested from a tobacco plant at knee-high stage or later. The terms "plant having a converter phenotype" and "converter plant" are used interchangeably herein. Similarly, a tobacco plant having a non-converter phenotype is a tobacco plant having a percent nicotine demethylation of less than 5% *(e.g.,* 4.9%, 4.5%, 4.2%, 4%, 3.8%, 3.5%, 3%, 2%, 1%, 0.8%, 0.6%, 0.5%, 0.05%, 0.02%, 0.01%, or undetectable) as measured in an ethylene-treated middle position leaf harvested from a tobacco plant at knee-high stage or later. The terms "plant having a non-converter phenotype" and "non-converter plant" are used interchangeably herein.

Nicotine and nornicotine can be measured in ethylene-treated leaves using methods known in the art (*e.g*., gas chromatography). Percent nicotine demethylation in a sample is calculated by dividing the level of nornicotine by the combined level of nicotine and nornicotine as measured in the sample, and multiplying by 100.

A plant comprising a mutation in a nicotine demethylase gene can be identified by selecting or screening the mutagenized plant material, or progeny thereof. Such screening and selection methodologies are known to those having ordinary skill in the art. Examples of screening and selection methodologies include, but are not limited to, Southern analysis, or PCR amplification for detection of a polynucleotide; Northern blots, S1 RNase protection, primer-extension, or RT-PCR amplification for detecting RNA transcripts; enzymatic assays for detecting enzyme or ribozyme activity of polypeptides and polynucleotides; and protein gel electrophoresis, Western blots, immunoprecipitation, and enzyme-linked immunoassays to detect polypeptides. Other techniques such as *in situ* hybridization, enzyme staining, and immunostaining also can be used to detect the presence or expression of polypeptides and/or polynucleotides. Methods for performing all of the referenced techniques are known.

A population of plants can be screened and/or selected for those members of the population that have a desired trait or phenotype conferred by a mutation in a nicotine demethylase gene, such as a non-converter phenotype. Selection and/or screening can be carried out over one or more generations, which can be useful to identify those plants that have a desired trait. In some embodiments, plants having a non-converter phenotype can be identified in the M₁ generation. Selection and/or screening can also be carried out in more than one geographic location. In addition, selection and/or screening can be carried out during a particular developmental stage in which the phenotype is exhibited by the plant.

A population of plants having a non-converter phenotype can be used to select and/or screen for plants with a reduced reversion rate, i.e., the percentage of converter phenotype plants in the next generation progeny of a non-converter plant. Reversion rate is measured by collecting seeds produced by a non-converter plant after self-pollination, planting 300 to 500 of the seeds, and determining the number of resulting plants having a converter phenotype. The reversion rate is expressed as the percentage of progeny plants that have a converter phenotype.

A non-converter plant having a mutation in a nicotine demethylase gene and exhibiting a reduced reversion rate can be bred to generate one or more tobacco hybrids, varieties or lines having a reversion rate that is statistically significantly less than the reversion rate of a control tobacco hybrid, variety or line having the same or similar genetic background, but carrying a wild type nicotine demethylase gene. Typically, a reduction in the reversion rate relative to a control hybrid, variety or line is considered statistically significant at p ≤ 0.05 with an appropriate parametric or non-parametric statistic, *e.g*., Chi-square test, Student's t-test, Mann-Whitney test, or F-test. In some embodiments, a reduction in the reversion rate is statistically significant at p < 0.01, p < 0.005, or p < 0.001.

The extent to which reversion rate is reduced typically depends on the tobacco type. For example, a non-converter Burley type tobacco having a mutation in a nicotine demethylase gene typically has a reversion rate that is reduced 10X or more (*e.g*., 10X to 1000X, 10X to 100X, 50X to 250X, 50X to 100X, 150X to 300X, 100X to 1000X, 500X to 1000X, 800X to 5000X, or 1500X to 10000X) relative to a Burley type tobacco variety of the same or similar genetic background, but having a wild type nicotine demethylase gene. In another example, a non-converter dark type tobacco having a mutation in a nicotine demethylase gene typically has a reversion rate that is reduced 2X or more (*e.g*., 2X to 100X, 2X to 5X, 2X to 10X, 5X to 30X, 10X to 50X, 5X to 100X, 10X to 100X, 50X to 300X, 250X to 500X, 300X to 3000X, or 3000X to 5000X) relative to a dark type tobacco variety of the same or similar genetic background, but having a wild type nicotine demethylase gene. In another example, a non-converter flue-cured type tobacco having a mutation in a nicotine demethylase gene typically has a reversion rate that is reduced 2X or more (*e.g*., 2X to 10X, 5X to 30X, 10X to 50X, 10X to 100X, 50X to 150X, 100X to 500X, 200X to 800X, 400X to 1000X, 500X to 3000X, or 1000X to 5000X) relative to a flue-cured type tobacco variety of the same or similar genetic background, but having a wild type nicotine demethylase gene. In some cases, the reversion rate of tobacco hybrids, varieties or lines comprising plants having a mutation in a nicotine demethylase gene can be so low as to be undetectable.

The method of screening for reduced reversion rate can depend on the source of the mutagenized plant material. For example, if the mutagenized plant material is seed from a plant having a converter phenotype, suitable methods of screening include identifying progeny having a mutation in a nicotine demethylase gene and/or identifying progeny having a non-converter phenotype. Once such progeny are identified, they are screened for those plants whose progeny exhibit a reduced reversion rate. In another example, if the mutagenized plant material is seed from a plant having a non-converter phenotype, a suitable method of screening includes identifying progeny having a mutation in a nicotine demethylase gene and/or determining whether progeny have a reduced reversion rate.

In some embodiments of methods described herein, lines resulting from breeding and screening for variant nicotine demethylase genes are evaluated in the field using standard field procedures. Control genotypes including the original unmutagenized parent are included and entries are arranged in the field in a randomized complete block design or other appropriate field design. Standard agronomic practices for tobacco are used, for example, the tobacco is harvested, weighed, and sampled for chemical and other common testing before and during curing. Statistical analyses of the data are performed to confirm the similarity of the selected lines to the parental line.

### Utility

Mutant and transgenic tobacco plants provided herein have particular uses in agricultural industries. Such a plant can be used in a breeding program as described herein to produce a tobacco line, variety or hybrid comprising plants having a non-converter phenotype, wherein the line, variety or hybrid has a reduced reversion rate as compared to a corresponding tobacco line, variety or hybrid that is wild type for the nicotine demethylase gene. In some cases, the mutant or transgenic tobacco plants provided herein can be crossed to plants having another desired trait to produce tobacco varieties having both a reduced reversion rate and another desired trait. Examples of other desired traits include drought tolerance, disease resistance, nicotine content, sugar content, leaf size, leaf width, leaf length, leaf color, leaf reddening, internode length, flowering time, lodging resistance, stalk thickness, leaf yield, disease resistance; high yield; high grade index; curability; curing quality; mechanical harvestability; holding ability; leaf quality; height; maturation; stalk size; and leaf number per plant. Tobacco lines, varieties or hybrids can be bred according to standard procedures in the art.

In other cases, based on the effect of disclosed nicotine demethylase mutations on the phenotype of plants having such mutations, one can search for and identify tobacco plants carrying in their genomes naturally occurring mutant alleles in a nicotine demethylase locus. Such plants can be used in a breeding program to produce a tobacco line, variety or hybrid comprising plants having a mutation in a nicotine demethylase gene, such a line, variety or hybrid having a reduced reversion rate as compared to a corresponding tobacco line, variety or hybrid having a wild type nicotine demethylase gene.

Tobacco plants having a mutation in a nicotine demethylase gene can be used to produce tobacco material for use in making tobacco products. Suitable tobacco material includes whole leaf, tobacco fines, tobacco dust, sized tobacco lamina, cut or roll pressed tobacco stem, volume expanded tobacco and shredded tobacco. Tobacco material from the disclosed mutant tobacco plants can be cured using curing methods known in the art such as air curing, fire curing, flue curing (*e.g*., bulk curing), and sun curing. In some embodiments, tobacco material is conditioned and/or fermented. See, e.g., U.S. Patent Publication No. 20050178398.

Tobacco plants having a mutation in a nicotine demethylase gene can be used to make a tobacco product having a reduced nornicotine content as compared to a corresponding product comprising tobacco obtained from a corresponding tobacco line, variety or hybrid comprising plants comprising a wild type nicotine demethylase gene. Tobacco products having a reduced amount of nitrosamine content can be manufactured using tobacco plant material described herein. The tobacco product typically has a reduced amount of nornicotine of less than about 5 mg/g. For example, the nornicotine content, or the NNN content, in such a product can be 4.5 mg/g, 4.0 mg/g, 3.5 mg/g, 3.0 mg/g, 2.5 mg/g, 2.0 mg/g, 1.5 mg/g, 1.0 mg/g, 750 µg/g, 500 µg/g, 250 µg/g, 100 µg/g, 75 µg/g, 50 µg/g, 25 µg/g, 10 µg/g, 7.0 µg/g, 5.0 µg/g, 4.0 µg/g, 2.0 µg/g, 1.0 µg/g, 0.5 µg/g, 0.4 µg/g, 0.2 µg/g, 0.1 µg/g, 0.05 µg/g, or 0.01 µg/g. The percentage of secondary alkaloids relative to total alkaloid content contained therein is less than 90%, e.g., less than 70%, 50%, 30%, 10%, 5%, 4%, 3%, 2%, 1.5%, 1%, 0.75%, 0.5%, 0.25%, or 0.1%.

The phrase "a reduced amount" with respect to nornicotine or NNN refers to an amount of nornicotine or NNN or both in a tobacco plant or plant component or a tobacco product that is less than what would be found in a wild-type tobacco plant or plant component or tobacco product from the same variety of tobacco, processed in the same manner, which was not made transgenic for reduced nornicotine or NNN or does not have a mutation in a nicotine demethylase gene. In one example, a wild-type tobacco plant of the same variety that has been processed in the same manner is used as a control to measure whether a reduction of nornicotine or NNN or both has been obtained by the methods described herein. In another example, plants having a reduced amount of nitrosamine content are evaluated using standard methods, for instance, by monitoring the presence or absence of a gene or gene product, e.g., a nicotine demethylase, a transgene, or a particular mutation in a gene. In still another example, nitrosamine content of plants resulting from a breeding program are compared to the nitrosamine content of one of the parent lines used to breed the plant having the reduced amount of nitrosamine. Levels of nornicotine and NNN or both are measured according to methods well known in the tobacco art.

Tobacco material obtained from the tobacco plants provided herein can be used to make tobacco products including, without limitation, cigarette products (*e.g*., cigarettes and bidi cigarettes), cigar products (*e.g*., cigar wrapping tobacco and cigarillos), pipe tobacco products, smokeless cigarette products, smokeless tobacco products (*e.g*., moist snuff, dry snuff, and chewing tobacco), films, chewables, tabs, shaped parts, gels, consumable units, insoluble matrices, hollow shapes and the like. See, e.g., U.S. Patent Publication No. US 2006/0191548.

The invention will be further described in the following examples, which do not limit the scope of the invention described in the claims.

### EXAMPLES

### Example 1 - Production of mutant Nicotiana plants

One gram of tobacco TN90 (Tennessee 90) converter seed (approximately 10,000 seeds) was washed in 0.1% Tween® for fifteen minutes and then soaked in 30 ml of ddH₂O for two hours. One hundred fifty (150) µl (0.5%) of EMS (Sigma Catalogue No. M-0880) was then mixed into the seed/ddH₂O solution and incubated for 8-12 hours (rotating at 30 rpm) under a hood at room temperature (RT, approximately 20°C). The liquid was then removed from the seeds and the liquid was mixed into 1 M NaOH overnight for decontamination and disposal. The seeds were then washed twice with 100 ml ddH₂O for 2-4 hours. The washed seeds were then suspended in 0.1% agar:water solution.

The EMS treated seeds in agar solution were evenly spread onto water soaked Carolina's Choice Tobacco Mix^{™} (Carolina Soil Company, Kinston, NC) in flats at a rate of -2000 seeds/flat. The flats were then covered by Saran^{™} wrap and placed in a growth chamber. Once the seedlings emerged from the soil, the Saran^{™} wrap was punctured to allow humidity to decline gradually. The Saran^{™} wrap was removed completely after two weeks. Flats were moved to a greenhouse and fertilized with NPK fertilizer. The seedlings were plugged into a float tray and grown until transplanting size. The plants were transplanted into a field. During growth, the plants were self-pollinated to form M₁ seeds. At the mature stage, five capsules were harvested from each of around 7000 plants and individual designations were given to the set of seeds from each plant. This formed the M₁ population.

### Example 2 - Identifcation of Mutations

A composite of M₁ seed from each M₀ plant of Example 1 was grown, leaves from 4 to 5 M₁ plants were pooled and DNA was extracted from the pooled tissue samples. Two pooled samples were taken from each M₁ line. DNeasy plant mini kits (QIAGEN, Catalogue no. 69104) were used for DNA extraction, following the manufacturer's manual.

IRDye™ 700-labeled forward primers and IRDye™ 800-labeled reverse primers were designed to amplify a nicotine demethylase gene (SEQ ID NO:1). Two pairs of sequence specific primers, which covered two separate exons, were selected to amplify the nicotine demethylase (ND) gene by PCR. Primers F6 (5'-GGAATTATGCCCATCCTACAG) and R1 (5'-CCAGCATTGCAGGGTTCGGGAAGA) covered the ND gene from -82 to +1139 and generated a 1,220 nucleotide fragment. Primers F3 (5'-CAGGTAAGGTCTAAAACGTGTGTTTGCTT) and R2 (5'-AATAAAGCTCAGGTGCCAGGCGAGGCGCTAT) covered the ND gene from +1720 to +2549 and generated an 830 nucleotide fragment.

Forward primers were prepared by mixing (1:4) IRDye™ 700-labeled primer:unlabeled primer with a concentration of 5 µM. Reverse primers were prepared by mixing (3:2) IRDye™ 800-labeled primer:unlabeled primer with a concentration of 5 µM. Stocked primers were prepared at 2:1 of Fwd:Rev ratio (5 µM total primer concentration). PCR amplification of the target region was done using 50-100 ng genomic DNA from pooled plant tissue DNA samples (in 10 µl reaction with 2 µl primer) and Platinum Taq DNA polymerase (Invitrogen, Catalogue no. 10966-034). PCR conditions were as follows: 1 cycle of 94°C for two minutes, 40 cycles of 94°C for one minute, 67°C for one minute, 72°C for 1.5 minutes, 1 cycle of 72°C for ten minutes, and hold at 4°C. Following amplification, samples were heat denatured and reannealed (1 cycle of 95°C for ten minutes, 95°C to 85°C at -2°C/second, and 85°C to 25°C at 0.1°C/second) to generate heteroduplexes between mutant amplicons and their wild-type counterparts.

Surveyor^{™} nuclease (Transgenomic®, Catalogue no. 706025) was used in accordance with kit recommendations to digest heteroduplexes. Nuclease incubation condition was 42°C for twenty minutes and reactions were stopped by the addition of Stop Solution (Transgenomic® kit). Heteroduplexes were denatured with sequencing loading dye (98% deionized formamide, 10 mM EDTA (pH 8.0), 0.025% bromophenol blue) by heating 95 ° C for two minutes. Denatured samples were chilled on ice and applied to denaturing polyacrylamide gel electrophoresis system. Electrophoresis was performed with a 6.5% KBPlus gel, run in a 18 cm plate assembly with 0.25 mm spacers on a LI-COR® DNA Analyzer (LI-COR® Biosciences) with running conditions of 1500-2000 V, 30 mA, 50 W and 45°C for 3.5 hours.

In the polyacrylamide gel lanes that had a mutation in the pool, a band was visible below the wild type band on the IRDye™ 700 infrared dye image. A counterpart band was visible in the same lane on the IRDye™ 800 infrared dye image. This band was the cleavage product labeled with IRDye™ 800 infrared dye from the complementary DNA strand. The sum of the length of the two counterpart bands was equal to the size of the amplicon. After image analysis, the mutation pools (with deferred bands) were identified.

A second round of screening was performed on individual plants from pools in which a mutation was detected. Individual plant DNA from positive lines was extracted and combined with wild type DNA samples for the second round of screening. This helped to separate wild type and mutant plants (including homozygous and heterozygous mutants) within same M₁ pool. Samples with cleaved bands had a mutant ND gene sequence, while samples lacking a cleaved band had a wild type ND gene sequence.

A third round of screening was used to distinguish heterozygous from homozygous plants by using only mutant plant DNA as a template. The samples with no cleaved bands were homozygous. Sequence trace information was analyzed using the CEQ 8000 sequencer (Beckman, Fullerton, California) to confirm the mutation. Using extracted DNA as the template, PCR amplification was performed to generate ND gene fragments for sequencing. PCR products were separated on a 1% agarose gel, purified, and sequenced.

The sequencing procedure was as follows: DNA was denatured by heating at 95° C for 2 minutes, and subsequently placed on ice. The sequencing reaction was prepared on ice using 0.5 to 10 µl of denatured DNA template, 2 µl of 1.6 pmole of the forward primer, 8 µl of DTCS Quick Start Master Mix and the total volume brought to 20 µl with water. The thermocycling program consisted of 30 cycles of the follow cycle: 96° C for 20 seconds, 50° C for 20 seconds, and 60° C for 4 minutes followed by holding at 4° C. The sequence was stopped by adding 5 µl of stop buffer (equal volume of 3M NaOAc and 100 mM EDTA and 1 µl of 20 mg/ml glycogen). The sample was precipitated with 60 µl of cold 95% ethanol and centrifuged at 6000g for 6 minutes. Ethanol was discarded. The pellet was 2 washes with 200 µl of cold 70% ethanol. After the pellet was dry, 40 µl of SLS solution was added and the pellet was resuspended and overlaid with a layer of mineral oil. The sample was then placed sequenced (CEQ 8000 Automated Sequencer). The sequences were aligned with wild type sequence. In addition, the genomic nicotine demethylase DNA for several selected lines was sequenced to confirm that only single mutation for nicotine demethylase gene was present in each line.

After screening 700 independent M₁ pools, 19 mutated lines were identified. The mutation in each line is set forth in Table 1.

**Table 1**

| **Nicotine Demethylase Gene Mutations in EMS Mutated Tobacco (TN90)** | | | |
|---|---|---|---|
| TOBACCO LINE | POSITION CHANGE¹ | CONTENT CHANGE | NOTE |
| TN90-4246 | +1985 nt from ATG +329 aa from M | G to A W329 Stop | Generated 329 aa nonsense mutation |
| TN90-1849 | +320 nt from ATG +107 aa from M | CtoT P107L | Missense mutation in SRS-1 domain |
| TN90-1394 | +412 nt from ATG +138 aa from M | G to A V138I | Missense mutation |
| TN90-1761A | +934 nt from ATG +312 aa from ATG | GtoA V312M | Missense mutation just before intron, in SRS-4 |
| TN90-4281 | +2191 nt from ATG +398 aa from M | G to A S 398 N | Missense mutation |
| TN90-1516 | +2307 nt from ATG +437 aa from M | G to A D437N | Missense mutation |
| TN90-1514 | +2307 nt from ATG +437 aa from M | GtoA D437N | Missense mutation |
| TN90-3320 | +437 nt from ATG +146 aa from M | GtoA S146N | Missense mutation |
| TN90-3341 | +704 nt from ATG +235 aa from M | CtoT P235L | Missense mutation |
| TN90-3387 | +668 nt from ATG +230 aa from M | G to A D230N | Missense mutation |
| TN90-1804 | +244 nt from ATG +82 aa from M | CtoT L82F | Missense mutation |
| TN90-1777 | +114 nt from ATG no aa change | CtoT P38P | Silent mutation |
| TN90-1803 | +342 nt from ATG no aa change | CtoT Y114Y | Silent mutation |
| TN90-4264 | +486 nt from ATG +163 aa from M | CtoT S162S | Silent mutation |
| TN90-1921 | +2024 nt from ATG +343 aa from M | GtoA K343K | Silent mutation |
| TN90-3147 | +429 nt from ATG no aa change | CtoT L142L | Silent mutation |
| TN90-4278 | +2021 nt from ATG +342 aa no change | G to A T342T | Silent mutation |
| TN90-4215 | +2291 nt from ATG +431 aa no change | GtoA E431E | Silent mutation |
| TN90-1431 | +2397 nt from ATG +467 aa from M | G to A E467K | Missense mutation |

| | | | |
|---|---|---|---|
| ¹nt = nucleotide number and aa = amino acid residue number in SEQ ID NOS: 1 and 2. | | | |

These mutated lines included one line with a truncated protein (TN90-4246), eleven lines with single amino acid changes (TN90-1849, TN90-1394, TN90-1761, TN90-4281, TN90-1516, TN90-1514, TN90-3320, TN90-3341, TN90-3387, TN90-1804, and TN90-1431) and seven lines with silent mutations (TN90-1777, TN90-1803, TN90-4264, TN90-1921, TN90-3147, TN90-4278, and TN215). These lines were transplanted into a field for further characterization. Additional M₁ seeds from the same lines set forth in Table 1 were seeded and grown in the greenhouse to screen for more homozygous plants and for analysis of alkaloid content.

### Example 3 - Measurement of Nicotine Demethylation

### Plant materials and induction treatment

The selected M₁ mutant lines of Example 2 grown in the field were tested for their ability to convert nicotine to nornicotine. A middle position leaf from each M₁ plant at knee-high stage or later was sprayed with a 0.3% ethylene solution (Prep brand Ethephon (Rhone-Poulenc)) to induce nornicotine formation. Each sprayed leaf was hung in a plastic covered curing rack equipped with a humidifier. Sampled leaves were sprayed periodically with the ethylene solution throughout the treatment period. Approximately three days after the ethylene treatment, leaves were collected and dried in a oven at 50°C for gas chromatographic (GC) analysis of alkaloids.

### Gas chromatographic alkaloid analysis

GC alkaloid analysis was performed as follows: samples (0.1 g) were shaken at 150 rpm with 0.5 ml 2N NaOH, and a 5 ml extraction solution which contained quinoline as an internal standard and methyl t-butyl ether. Samples were analyzed on an HP 6890 GC (Hewlett Packard, Wilmington, DE, USA) equipped with a FID detector. A temperature of 250°C was used for the detector and injector. An GC column (30m-0.32nm-1 m) consisting of fused silica cross-linked with 5% phenol and 95% methyl silicon was used at a temperature gradient of 110-185°C at 10°C per minute. The column was operated at a flow rate at 100°C at 1.7 cm³/min with a split ratio of 40:1 with a 2 µl injection volume using helium as the carrier gas. Percent nicotine demethylation was calculated as the amount of nicotine divided by the sum of the amounts of nicotine and nornicotine, multiplied by 100.

Table 2 shows the percent of plants having a non-converter phenotype and the mean percent nicotine demethylation for eight mutant lines, in relation to the genetic mutation status of individual plants of that line, including homozygous mutant, heterozygous mutant, and homozygous wild type. Four of the mutant lines had a percent nicotine demethylation of less than 5% in the M₁ generation and were classified as exhibiting a non-converter phenotype, mutant lines 4246, 1849, 4215 and 4278. The other four mutant lines had a percent nicotine demethylation of 5% or greater in the M₁ generation and were classified as having a converter phenotype, mutant lines 1394, 3320, 4264 and 1924.

**Table 2**

| **Nicotine Demethylation Levels in Nicotine Demethylase Mutant Lines** | | | | | |
|---|---|---|---|---|---|
| EMS Line (TN90) | Status | Number of Plants | Mean % Nicotine Demethylation | % Converter Phenotype | % Non-converter Phenotype |
| 4246 | Homozygous | 11 | 0.85 | 0 | 100 |
| | Heterozygous | 36 | 51.47 | 94.45 | 5.6 |
| | Wild Type | 34 | 68.85 | 100 | 0 |
| | Total | 81 | | | |
| 1849 | Homozygous | 2 | 0.65 | 0 | 100 |
| | Heterozygous | 21 | 62.28 | 95.2 | 4.8 |
| | Wild Type | 2 | 71.2 | 100 | 0 |
| | Total | 25 | | | |
| 4215 | Homozygous | 4 | 0.025 | 0 | 100 |
| | Heterozygous | 12 | 43.32 | 100 | 0 |
| | Wild Type | 5 | 79.66 | 100 | 0 |
| | Total | 21 | | | |
| 4278 | Homozygous | 6 | 1.05 | 0 | 100 |
| | Heterozygous | 12 | 34.3 | 83.3 | 16.7 |
| | Wild Type | 2 | 82.1 | 100 | 0 |
| | Total | 20 | | | |
| 1394 | Homozygous | 1 | 96.8 | 100 | 0 |
| | Heterozygous | 2 | 88.6 | 100 | 0 |
| | Wild Type | 3 | 65.77 | 100 | 0 |
| | Unknown | 7 | 66.59 | 85.7 | 14.3 |
| | Total | 13 | | | |
| 3320 | Homozygous | 4 | 62.54 | 100 | 0 |
| | Heterozygous | 9 | 48.55 | 100 | 0 |
| | Wild Type | 6 | 62.03 | 100 | 0 |
| | Total | 19 | | | |
| 4264 | Homozygous | 2 | 83.6 | 100 | 0 |
| | Heterozygous | 3 | 59.27 | 100 | 0 |
| | Wild Type | 4 | 31.48 | 100 | 0 |
| | Total | 9 | | | |
| 1921 | Homozygous | 1. | 5.7 | 100 | 0 |
| | Heterozygous | 10 | 38.9 | 100 | 0 |
| | Wild Type | 0 | -- | -- | -- |
| | Total | 11 | | | |

Figures 1A-1D show the frequency of converter and non-converter phenotypes among heterozygous mutant, homozygous mutant and homozygous wild-type M₁ plants for the mutant lines 4246, 1849,4215, and 4278. Figures 1E and 1F show representative results for mutant lines in which there was no difference in nicotine demethylation among M₁ plants.

### Example 4 - RNA Expression Analysis in Nicotine Demethylase Mutant Lines

RNA from two lines was analyzed using semi-quantitative RT-PCR to measure their mRNA expression. About 20 individual M₁ plants from each line were ethylene treated as described in Example 3, and total RNA was extracted 3 days post-treatment. Total RNA was isolated using RNeasy Plant Mini Kit® (Qiagen, Inc., Valencia, California) following the manufacturer's protocol. The tissue sample was ground under liquid nitrogen to a fine powder using a DEPC-treated mortar and pestle. Approximately 100 mg of ground tissue was transferred to a sterile 1.5 ml Eppendorf tube® and the tube placed in liquid nitrogen until all samples were collected. Then, 450 µl of Buffer RLT as provided in the kit (with the addition of β-Mercaptoethanol) was added to each individual tube. The samples were vortexed vigorously and incubated at 56°C for three minutes. The lysate was then applied to the QIAshredder^{™} spin column sitting in a 2-ml collection tube, and centrifuged for two minutes at maximum speed.

The flow through was collected and 0.5 volume of ethanol was added to the cleared lysate. The sample was mixed well and transferred to an Rneasy® mini spin column sitting in a 2 ml collection tube. The sample was centrifuged for one minute at 10,000 rpm. Next, 700µl of buffer RW1 was pipetted onto the Rneasy® column and centrifuged for one minute at 10,000 rpm. Buffer RPE was pipetted onto the Rneasy® column in a new collection tube and centrifuged for one minute at 10,000 rpm. Buffer RPE was again added to the Rneasy® spin column and centrifuged for two minutes at maximum speed to dry the membrane.

To eliminate any ethanol carry over, the membrane was placed in a separate collection tube and centrifuged for an additional one minute at maximum speed. The Rneasy® column was transferred into a new 1.5 ml collection tube, and 40 µl of Rnase-free water was pipetted directly onto the Rneasy® membrane. This final elute tube was centrifuged for one minute at 10,000 rpm. Quality and quantity of total RNA was analyzed by denatured formaldehyde gel and spectrophotometer.

First strand cDNA was produced using SuperScript^{™} reverse transcriptase following manufacturer's protocol (Invitrogen, Carlsbad, California). About 100 ng of total RNA was used for first strand cDNA generation.

RT-PCR was carried out with 100 pmoles each of forward and reverse primers. Reaction conditions were 94°C for two minutes and then 40 cycles of PCR at 94°C for one minute, 67°C for one minute, 72°C for three minutes, followed by a single extension at 72°C for ten minutes. Fifty microliters of the amplified sample were analyzed by electrophoresis using a 1% agarose gel.

The agarose gels were stained using ethidium bromide and the amount of ND RNA present was classified as low or high based on band intensity. Selected samples were sliced and purified from the agarose gel. The purified DNA was sequenced by CEQ 8000 as described above.

### Example 5 -Nicotine Demethylase Sequence Analysis

The amino acid sequence set forth in SEQ ID NO:2 was subjected to analysis using the TFSEARCH program (cbrc.jp/htbin/nph-tfsearch) and the Web Signal Scan Program (dna.affrc.go.jp/sigscan) to identify regulatory region elements (*e.g*., TATA and CAAT boxes), organ-specific elements, and WRKY elements. As shown in Figure 2, the analysis indicated that SEQ ID NO:2 contains six substrate recognition sites (SRS) at amino acids 108-129, 212-220, 249-256, 312-326, 380-390, and 491-497, an N-terminal hydrophobic transmembrane domain at amino acids 9-20, a proline-rich region at amino acids 34-38, a threonine-containing oxygen-binding pocket at amino acids 346-351, a K-helix consensus at amino acids 353-356, a PERF consensus at amino acids 430-433, and a cysteine-containing heme-binding region at amino acids 450-459.

### Example 6 - Nicotine Conversion Stability in Nicotine Demethylase Mutant Lines

Large scale field trials were conducted with selected M₂ mutant lines, 4246-8 and 1859-8B, using a screened low converter (LC) Certified commercial variety (TN90-LC) and its high converter counterpart (TN90-C) as controls. The screened LC Certified variety is commercially available from F.W. Rickard Seeds (Winchester, Kentucky). Screened LC Certified seed is collected from plants grown from screened LC Foundation seed. Screened LC Foundation seed is collected from a population of tobacco plants from which plants with a nicotine conversion level higher than 3% were removed, and from which any flowers or capsules that were produced prior to removing the plants having a nicotine conversion level higher than 3% were removed.

Two M₂ mutant lines, 4246-8 and 1859-8B, were produced through self pollination of M₁ homozygous mutant plants. These lines were grown in 3 field trials with total population of about 200 plants per line. The plants grown in the field were tested for their ability to convert nicotine to nornicotine. A middle position leaf from each M₂ plant was ethylene treated as described in Example 3. Approximately three days after the ethylene treatment, leaves were collected and dried in an oven at 50°C for gas chromatographic (GC) analysis of alkaloids as described in Example 3.

Table 3 shows the nicotine conversion stability in M₂ mutant lines in comparison of commercial LC line and converter control. Mutant line 4246-8 had mean percentage nicotine conversion of 1.9% and had no plants that were classified as high converter. Mutant line 1849-8B had mean percentage conversion of 2.1% and had 3 plants from total of 214 that were classified as high converters. The LC and converter lines had mean conversion of 6.6% and 80.6%, respectively, and had 24% and 100% of the plants, respectively, classified as high converters.

The M₃ generation had a similar low frequency of conversion, demonstrating the stability of the low-converter phenotype in the mutant lines.

**Table 3**

| **Nicotine Demethylation Levels in Nicotine Demethylase Mutant M₂ Lines, Screened Low Converter and Converter Controls** | | | | |
|---|---|---|---|---|
| Line | Number of Plants | Number of Converters | Converters (% of Population) | Average Conversion Rate (%) |
| 4246-8 | 184 | 0 | 0 | 1.9 |
| 1849-8B | 212 | 3 | 1.4 | 2.1 |
| TN90-LC | 218 | 53 | 24.3 | 6.6 |
| TN90-C | 95 | 95 | 100 | 80.6 |

### Example 7 -Detection of Tobacco Specific Nitrosamine Formation in Nicotine Demethylase Mutant Lines

Large scale field trials were conducted with selected M₂ mutant lines, 4246-8 and 1849-8B, using screened low converter (LC) Certified commercial variety (TN90-LC) and its high converter counterpart (TN90-C) as controls as described in Example 6.

The field grown plants from Example 6 were grown to maturity, and were harvested and air-cured using standard tobacco production practices. The tobacco chemistry was analyzed by gas-chromatographic-TAE analysis. Table 4 depicts the tobacco specific nitrosamine (TSNA) content of mutant lines in comparison to LC and converter controls. The N-nitrosonornicotine (NNN) content, which is directly derived from nornicotine, and total TSNA content in mutant lines were lower than those in TN90-LC and TN90-converter lines.

**Table 4**

| **N-nitrosonornicotine (NNN) and Total Tobacco Specific Nitrosamine (TSNA) Levels in Air-cured Burley Tobacco Mutant Lines** | | |
|---|---|---|
| Line | NNN (ppm) | Total TSNA (ppm) |
| 4246-8 | 0.7 | 0.9 |
| 1849-8B | 0.8 | 0.9 |
| TN 90 Empty Vector | 1.6 | 1.9 |
| TN 90-LC | 1.6 | 1.6 |
| TN 90-C (high converter) | 5.5 | 5.6 |

### Example 8 -Phenotypic Characteristics in Nicotine Demethylase Mutant lines

Large scale field trials of nicotine demethylase mutant lines were grown to maturity as described in Example 7. Plant height, leaf length, leaf width, and yield were measured. The results are shown in Table 5.

**Table 5.**

| **Phenotypic Characteristics of Burley Tobacco Mutant Lines** | | | | | |
|---|---|---|---|---|---|
| **Line** | **Plant height-topped (cm)** | **Plant height-not topped (cm)** | **10^{th} leaf length (cm)** | **10^{th} leaf width (cm)** | **Yield (lbs/acre)** |
| 4246-8 | 118 | 146 | 62 | 32 | 2904 |
| 1849-8B | 112 | 138 | 60 | 34 | 2944 |
| TN90 LC | 138 | 179 | 70 | 39 | 3361 |
| TN90 C | 141 | 182 | 69 | 40 | 3175 |

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. A tobacco plant having a mutation in the amino acid sequence set forth in SEQ ID NO:2, said plants exhibiting a non-converter phenotype, wherein said mutation is selected from the group consisting of
a) a proline at amino acid 107 of SEQ ID NO:2 replaced with an amino acid selected from the group consisting of alanine, arginine, asparagine, aspartic acid, cysteine, glutamic acid, glutamine, histidine, isoleucine, leucine, lysine, methionine, phenylalanine, serine, threonine, tryptophan, tyrosine, and valine; and
b) a deletion at P107 relative to SEQ ID NO:2.

2. The tobacco plant of claim 1, wherein said mutation is
a proline at amino acid 107 of SEQ ID NO:2 replaced with leucine.

3. The plant of any one of claims 1-2, wherein said plant is an F1 hybrid plant.

4. A method of making a mutant tobacco plant comprising:
a) introducing at least one mutation in cells of a *Nicotiana* species in an amino acid sequence set forth in SEQ ID NO:2, wherein said mutation is selected from the group consisting of
(i) a proline at amino acid 107 of SEQ ID NO:2 replaced with an amino acid selected from the group consisting of alanine, arginine, asparagine, aspartic acid, cysteine, glutamic acid, glutamine, histidine, isoleucine, leucine, lysine, methionine, phenylalanine, serine, threonine, tryptophan, tyrosine, and valine; and
(ii) a deletion at P107 relative to SEQ ID NO:2; and
b) identifying at least one plant obtained from said cells that contains said at least one mutation.

5. The method of claim 4, wherein said cells are in a seed.

6. The method of claim 4, wherein said mutation is
a proline at amino acid 107 of SEQ ID NO:2 replaced with leucine.

7. A progeny of a plant as defined in any one of claims 1-3 having the mutation as defined in claim 1 or 2.

## Patentansprüche

1. Tabakpflanze mit einer Mutation in der in SEQ ID NO: 2 dargelegten Aminosäuresequenz, wobei die Pflanze einen Nicht-Konverter-Phänotyp aufweist, wobei die Mutation ausgewählt ist aus der Gruppe bestehend aus
(a) einem Prolin an Aminosäure 107 von SEQ ID NO: 2, das mit einer Aminosäure ausgetauscht ist, ausgewählt aus der Gruppe bestehend aus Alanin, Arginin, Asparagin, Asparaginsäure, Cystein, Glutaminsäure, Glutamin, Histidin, Isoleucin, Leucin, Lysin, Methionin, Phenylalanin, Serin, Threonin, Tryptophan, Tyrosin und Valin; und
(b) einer Deletion an P107 in Bezug auf SEQ ID NO: 2.

2. Tabakpflanze nach Anspruch 1, wobei die Mutation ein durch Leucin ausgetauschtes Prolin an Aminosäure 107 von SEQ ID NO: 2 ist.

3. Pflanze nach einem beliebigen der Ansprüche 1-2, wobei die Pflanze eine F1-Hybrid-Pflanze ist.

4. Verfahren zum Herstellen einer mutierten Tabakpflanze, umfassend:
(a) Einführen mindestens einer Mutation in Zellen einer *Nicotiana-*Spezies in einer in SEQ ID NO: 2 dargelegten Aminosäuresequenz, wobei die Mutation ausgewählt ist aus der Gruppe bestehend aus:
(i) einem Prolin an Aminosäure 107 von SEQ ID NO: 2, das mit einer Aminosäure ausgetauscht ist, ausgewählt aus der Gruppe bestehend aus Alanin, Arginin, Asparagin, Asparaginsäure, Cystein, Glutaminsäure, Glutamin, Histidin, Isoleucin, Leucin, Lysin, Methionin, Phenylalanin, Serin, Threonin, Tryptophan, Tyrosin und Valin; und
(ii) einer Deletion an P107 in Bezug auf SEQ ID NO: 2; und
(b) Identifizieren mindestens einer aus den Zellen erhaltenen Pflanze, die die mindestens eine Mutation enthält.

5. Verfahren nach Anspruch 4, wobei die Zellen in einem Samen sind.

6. Verfahren nach Anspruch 4, wobei die Mutation ein durch Leucin ausgetauschtes Prolin an Aminosäure 107 von SEQ ID NO: 2 ist.

7. Nachkomme einer wie in einem beliebigen der Ansprüche 1-3 definierten Pflanze mit der wie in Anspruch 1 oder 2 definierten Mutation.

## Revendications

1. Plant de tabac présentant une mutation dans la séquence d'acides aminés présentée dans SEQ ID NO: 2, lesdits plants présentant un phénotype non convertisseur, dans lequel ladite mutation est choisie dans le groupe constitué par
a) une proline au niveau de l'acide aminé 107 de SEQ ID NO: 2 remplacée par un acide aminé choisi dans le groupe constitué par l'alanine, l'arginine, l'asparagine, l'acide aspartique, la cystéine, l'acide glutamique, la glutamine, l'histidine, l'isoleucine, la leucine, la lysine, la méthionine, la phénylalanine, la sérine, la thréonine, le tryptophane, la tyrosine et la valine ; et
b) une délétion au niveau de P107 par rapport à SEQ ID NO: 2.

2. Plant de tabac selon la revendication 1, dans lequel ladite mutation est une proline au niveau de l'acide aminé 107 de SEQ ID NO: 2 remplacée par une leucine.

3. Plant selon l'une quelconque des revendications 1 à 2, dans lequel ledit plant est un plant hybride F1.

4. Procédé de préparation d'un plant de tabac mutant, comprenant :
a) l'introduction d'au moins une mutation dans les cellules d'une espèce *Nicotiana* dans une séquence d'acides aminés présentée dans SEQ ID NO: 2, dans laquelle ladite mutation est choisie dans le groupe constitué par
(i) une proline au niveau de l'acide aminé 107 de SEQ ID NO: 2 remplacée par un acide aminé choisi dans le groupe constitué par l'alanine, l'arginine, l'asparagine, l'acide aspartique, la cystéine, l'acide glutamique, la glutamine, l'histidine, l'isoleucine, la leucine, la lysine, la méthionine, la phénylalanine, la sérine, la thréonine, le tryptophane, la tyrosine et la valine ; et
(ii) une délétion au niveau de P107 par rapport à SEQ ID NO: 2; et
b) l'identification d'au moins un plant obtenu à partir desdites cellules qui contient ladite au moins une mutation.

5. Procédé selon la revendication 4, dans lequel lesdites cellules sont dans une graine.

6. Procédé selon la revendication 4, dans lequel ladite mutation est une proline au niveau de l'acide aminé 107 de SEQ ID NO: 2 remplacée par une leucine.

7. Descendance d'un plant tel que défini selon l'une quelconque des revendications 1 à 3, présentant la mutation telle que définie selon la revendication 1 ou 2.


### SEQUENCE LISTING

<110> U.S. Smokeless Tobacco Company
<120> TOBACCO PLANTS HAVING A MUTATION IN A
   NICOTINE DEMETHYLASE GENE
<130> F66559PCEPT1
<150> US 11/611,782
   <151> 2006-12-15
<160> 11
<170> FastSEQ for windows version 4.0
   <210> 1
   <211> 2552
   <212> DNA
   <213> Nicotiana tabacum
<400> 1
   atgctttctc ccatagaagc cattgtagga ctagtaacct tcacatttct cttcttcttc 60
   ctatggacaa aaaaatctca aaaaccttca aaacccttac caccgaaaat ccccggagga 120
   tggccggtaa tcggccatct tttccacttc aatgacgacg gcgacgaccg tccattagct 180
   cgaaaactcg gagacttagc tgacaaatac ggccccgttt tcacttttcg gctaggcctt 240
   ccccttgtct tagttgtaag cagttacgaa gctgtaaaag actgtttctc tacaaatgac 300
   gccatttttt ccaatcgtcc agcttttctt tacggcgatt accttggcta caataatgcc 360
   atgctatttt tggccaatta cggaccttac tggcgaaaaa atcgaaaatt agttattcag 420
   gaagttctct ccgctagtcg tctcgaaaaa ttcaaacacg tgagatttgc aagaattcaa 480
   gcgagcatta agaatttata tactcgaatt gatggaaatt cgagtacgat aaatttaact 540
   gattggttag aagaattgaa ttttggtctg atcgtgaaga tgatcgctgg aaaaaattat 600
   gaatccggta aaggagatga acaagtggag agatttaaga aagcgtttaa ggattttatg 660
   attttatcaa tggagtttgt gttatgggat gcatttccaa ttccattatt taaatgggtg 720
   gattttcaag ggcatgttaa ggctatgaaa aggactttta aagatataga ttctgttttt 780
   cagaattggt tagaggaaca tattaataaa agagaaaaaa tggaggttaa tgcagaaggg 840
   aatgaacaag atttcattga tgtggtgctt tcaaaaatga gtaatgaata tcttggtgaa 900
   ggttactctc gtgatactgt cattaaagca acggtgtttg taagttcatc tgtcattttt 960
   catttattca cttttatttt gaggagcaga catgttaata ataatttgga gcaactgtaa 1020
   agttatctat gtgtacaggt tcgagcctca ggtgcaacca ctaatgcttg tattagatta 1080
   tgttgtctgc atcatacccc taattggagt gtggctcttc ccgaaccctg caatgctgga 1140
   tgctggatgc tttatgtatc agactgacct ttttgttaaa ctatctaaat actaaggatg 1200
   atttaataaa aatatagaat ggtaaacaga aaaagatgag attatttttg gggctatatg 1260
   gattcgcccg ggctttggga ggtaaaacgg tatctaccag ttgagacttt actccagaac 1320
   tttatctcga gagctctgaa taaaaatgaa atagtattta ccactccaaa atctttgatg 1380
   gtaaaaagat gagatataac ctcttataat tgattgaacc acgttgatag aataaaactt 1440
   ctttactccc attcagcata agaaaaatga aaccaaacgg aattcttctc ttttttaggg 1500
   ggaaattcct taattgcttg ttgaatatag attcatgtcg ttattctatt tttaataatg 1560
   atgaaaatca atatagtcaa agttaatact tatgtcattt ggtttgcgga caagttatat 1620
   tggaactata taatacgtct attatagaat agtgattatt tagaggatat acattttttt 1680
   tggataaata tttgatttat tggattaaaa atagaatata caggtaaggt ctaaaacgtg 1740
   tgtttgcttt tacactaaat aaacttgacc tcgtacaatt ctaagaaaat atttgaaata 1800
   aatgaattat tttattgtta atcaattaaa aaaatcatag tatagatgag atgtgtgcat 1860
   acttgacaat aactatacta actaaaacaa ggtatgtgaa taattgatat tcctttttta 1920
   attattcttt tttccagagt ttggtcttgg atgcagcaga cacagttgct cttcacataa 1980
   attggggaat ggcattattg ataaacaatc aaaaggcctt gacgaaagca caagaagaga 2040
   tagacacaaa agttggtaag gacagatggg tagaagagag tgatattaag gatttggtat 2100
   acctccaagc tattgttaaa gaagtgttac gattatatcc accaggacct ttgttagtac 2160
   cacacgaaaa tgtagaagat tgtgttgtta gtggatatca cattcctaaa gggacaagat 2220
   tattcgcaaa cgtcatgaaa ctgcaacgtg atcctaaact ctggtctgat cctgatactt 2280
   tcgatccaga gagattcatt gctactgata ttgactttcg tggtcagtac tataagtata 2340
   tcccgtttgg ttctggaaga cgatcttgtc cagggatgac ttatgcattg caagtggaac 2400
   acttaacaat ggcacatttg atccaaggtt tcaattacag aactccaaat gacgagccct 2460
   tggatatgaa ggaaggtgca ggcataacta tacgtaaggt aaatcctgtg gaactgataa 2520
   tagcgcctcg cctggcacct gagctttatt aa                                2552
<210> 2
   <211> 517
   <212> PRT
   <213> Nicotiana tabacum
<400> 2
   Met Leu Ser Pro Ile Glu Ala Ile Val Gly Leu Val Thr Phe Thr Phe
   1 5 10 15
   Leu Phe Phe Phe Leu Trp Thr Lys Lys Ser Gln Lys Pro Ser Lys Pro
   20 25 30
   Leu Pro Pro Lys Ile Pro Gly Gly Trp Pro Val Ile Gly His Leu Phe
   35 40 45
   His Phe Asn Asp Asp Gly Asp Asp Arg Pro Leu Ala Arg Lys Leu Gly
   50 55 60
   Asp Leu Ala Asp Lys Tyr Gly Pro Val Phe Thr Phe Arg Leu Gly Leu
   65 70 75 80
   Pro Leu Val Leu Val Val Ser Ser Tyr Glu Ala Val Lys Asp Cys Phe
   85 90 95
   Ser Thr Asn Asp Ala Ile Phe Ser Asn Arg Pro Ala Phe Leu Tyr Gly
   100 105 110
   Asp Tyr Leu Gly Tyr Asn Asn Ala Met Leu Phe Leu Ala Asn Tyr Gly
   115 120 125
   Pro Tyr Trp Arg Lys Asn Arg Lys Leu Val Ile Gln Glu Val Leu Ser
   130 135 140
   Ala Ser Arg Leu Glu Lys Phe Lys His Val Arg Phe Ala Arg Ile Gln
   145 150 155 160
   Ala Ser Ile Lys Asn Leu Tyr Thr Arg Ile Asp Gly Asn Ser Ser Thr
   165 170 175
   Ile Asn Leu Thr Asp Trp Leu Glu Glu Leu Asn Phe Gly Leu Ile Val
   180 185 190
   Lys Met Ile Ala Gly Lys Asn Tyr Glu Ser Gly Lys Gly Asp Glu Gln
   195 200 205
   Val Glu Arg Phe Lys Lys Ala Phe Lys Asp Phe Met Ile Leu Ser Met
   210 215 220
   Glu Phe Val Leu Trp Asp Ala Phe Pro Ile Pro Leu Phe Lys Trp Val
   225 230 235 240
   Asp Phe Gln Gly His Val Lys Ala Met Lys Arg Thr Phe Lys Asp Ile
   245 250 255
   Asp Ser Val Phe Gln Asn Trp Leu Glu Glu His Ile Asn Lys Arg Glu
   260 265 270
   Lys Met Glu Val Asn Ala Glu Gly Asn Glu Gln Asp Phe Ile Asp Val
   275 280 285
   Val Leu Ser Lys Met Ser Asn Glu Tyr Leu Gly Glu Gly Tyr Ser Arg
   290 295 300
   Asp Thr Val Ile Lys Ala Thr Val Phe Ser Leu Val Leu Asp Ala Ala
   305 310 315 320
   Asp Thr Val Ala Leu His Ile Asn Trp Gly Met Ala Leu Leu Ile Asn
   325 330 335
   Asn Gln Lys Ala Leu Thr Lys Ala Gln Glu Glu Ile Asp Thr Lys Val
   340 345 350
   Gly Lys Asp Arg Trp Val Glu Glu Ser Asp Ile Lys Asp Leu Val Tyr
   355 360 365
   Leu Gln Ala Ile Val Lys Glu Val Leu Arg Leu Tyr Pro Pro Gly Pro
   370 375 380
   Leu Leu Val Pro His Glu Asn Val Glu Asp Cys Val Val Ser Gly Tyr
   385 390 395 400
   His Ile Pro Lys Gly Thr Arg Leu Phe Ala Asn Val Met Lys Leu Gln
   405 410 415
   Arg Asp Pro Lys Leu Trp Ser Asp Pro Asp Thr Phe Asp Pro Glu Arg
   420 425 430
   Phe Ile Ala Thr Asp Ile Asp Phe Arg Gly Gln Tyr Tyr Lys Tyr Ile
   435 440 445
   Pro Phe Gly Ser Gly Arg Arg Ser Cys Pro Gly Met Thr Tyr Ala Leu
   450 455 460
   Gln Val Glu His Leu Thr Met Ala His Leu Ile Gln Gly Phe Asn Tyr
   465 470 475 480
   Arg Thr Pro Asn Asp Glu Pro Leu Asp Met Lys Glu Gly Ala Gly Ile
   485 490 495
   Thr Ile Arg Lys Val Asn Pro Val Glu Leu Ile Ile Ala Pro Arg Leu
   500 505 510
   Ala Pro Glu Leu Tyr
   515
<210> 3
   <211> 517
   <212> PRT
   <213> Nicotiana tabacum
<400> 3
   Met Leu Ser Pro Ile Glu Ala Ile Val Gly Leu Val Thr Phe Thr Phe
   1               5                   10                  15
   Leu Phe Phe Phe Leu Trp Thr Lys Lys Ser Gln Lys Pro Ser Lys Pro
               20                  25                  30
   Leu Pro Pro Lys Ile Pro Gly Gly Trp Pro Val Ile Gly His Leu Phe
           35                  40                  45
   His Phe Asn Asp Asp Gly Asp Asp Arg Pro Leu Ala Arg Lys Leu Gly
       50                  55                  60
   Asp Leu Ala Asp Lys Tyr Gly Pro Val Phe Thr Phe Arg Leu Gly Leu
   65                  70                  75                  80
   Pro Leu Val Leu Val Val Ser Ser Tyr Glu Ala Val Lys Asp Cys Phe
                   85                  90                  95
   Ser Thr Asn Asp Ala Ile Phe Ser Asn Arg Pro Ala Phe Leu Tyr Gly
               100                 105                 110
   Asp Tyr Leu Gly Tyr Asn Asn Ala Met Leu Phe Leu Ala Asn Tyr Gly
           115                 120                 125
   Pro Tyr Trp Arg Lys Asn Arg Lys Leu Val Ile Gln Glu Val Leu Ser
       130                 135                 140
   Ala Ser Arg Leu Glu Lys Phe Lys His Val Arg Phe Ala Arg Ile Gln
   145                 150                 155                 160
   Ala Ser Met Lys Asn Leu Tyr Thr Arg Ile Asp Gly Asn Ser Ser Thr
                   165                 170                 175
   Ile Asn Leu Thr Asp Trp Leu Glu Glu Leu Asn Phe Gly Leu Ile Val
               180                 185                 190
   Lys Met Ile Ala Gly Lys Asn Tyr Glu Ser Gly Lys Gly Asp Glu Gln
           195                 200                 205
   Val Glu Arg Phe Lys Lys Ala Phe Lys Asp Phe Met Ile Leu Ser Met
       210                 215                 220
   Glu Phe Val Leu Trp Asp Ala Phe Pro Ile Pro Leu Phe Lys Trp Val
   225                 230                 235                 240
   Asp Phe Gln Gly His Val Lys Ala Met Lys Arg Thr Phe Lys Asp Ile
                   245                 250                 255
   Asp Ser Val Phe Gln Asn Trp Leu Glu Glu His Ile Asn Lys Arg Glu
               260                 265                 270
   Lys Met Glu Val Asn Ala Glu Gly Asn Glu Gln Asp Phe Ile Asp Val
           275                 280                 285
   Val Leu Ser Lys Met Ser Asn Glu Tyr Leu Gly Glu Gly Tyr Ser Arg
       290                 295                 300
   Asp Thr Val Ile Glu Ala Thr Val Phe Ser Leu Val Leu Asp Ala Ala
   305                 310                 315                 320
   Asp Thr Val Ala Leu His Ile Asn Trp Gly Met Ala Leu Leu Ile Asn
                   325                 330                 335
   Asn Gln Lys Ala Leu Thr Lys Ala Gln Glu Glu Ile Asp Thr Lys Val
               340                 345                 350
   Cys Lys Asp Arg Trp Val Glu Glu Ser Asp Ile Lys Asp Leu Val Tyr
           355                 360                 365
   Leu Gln Ala Ile Val Lys Glu Val Leu Arg Leu Tyr Pro Pro Gly Pro
       370                 375                 380
   Leu Leu Val Pro His Glu Asn Val Glu Asp Cys Val Val Ser Gly Tyr
   385                 390                 395                 400
   His Ile Pro Lys Gly Thr Arg Leu Phe Ala Asn Val Met Lys Leu Gln
                   405                 410                 415
   Arg Asp Pro Lys Leu Trp Ser Asp Pro Asp Thr Phe Asp Pro Glu Arg
               420                 425                 430
   Phe Ile Ala Thr Asp Ile Asp Phe Arg Gly Gln Tyr Tyr Lys Tyr Ile
           435                 440                 445
   Pro Phe Gly Pro Gly Arg Arg Ser Cys Pro Gly Met Thr Tyr Ala Leu
       450                 455                 460
   Gln Val Glu His Leu Thr Met Ala His Leu Ile Gln Gly Phe Asn Tyr
   465                 470                 475                 480
   Arg Thr Pro Asn Asp Glu Pro Leu Asp Met Lys Glu Gly Ala Gly Ile
                   485                 490                 495
   Thr Ile Arg Lys Val Asn Pro Val Glu Leu Ile Ile Ala Pro Arg Leu
               500                 505                 510
   Ala Pro Glu Leu Tyr
           515
<210> 4
   <211> 517
   <212> PRT
   <213> Nicotiana tabacum
<400> 4
   Met Leu Ser Pro Ile Glu Ala Ile Val Gly Leu Val Thr Phe Thr Phe
   1               5                   10                  15
   Leu Phe Phe Phe Leu Trp Thr Lys Lys Ser Gln Lys Pro Ser Lys Pro
               20                  25                  30
   Leu Pro Pro Lys Ile Pro Gly Gly Trp Pro Val Ile Gly His Leu Phe
           35                  40                  45
   His Phe Asn Asp Asp Gly Asp Asp Arg Pro Leu Ala Arg Lys Leu Gly
       50                  55                  60
   Asp Leu Ala Asp Lys Tyr Gly Pro Val Phe Thr Phe Arg Leu Gly Leu
   65                  70                  75                  80
   Pro Leu Val Leu Val Val Ser Ser Tyr Glu Ala Val Lys Asp Cys Phe
                   85                  90                  95
   Ser Thr Asn Asp Ala Ile Phe Ser Asn Arg Pro Ala Phe Leu Tyr Gly
               100                 105                 110
   Asp Tyr Leu Gly Tyr Asn Asn Ala Met Leu Phe Leu Ala Asn Tyr Gly
           115                 120                 125
   Pro Tyr Trp Arg Lys Asn Arg Lys Leu Val Ile Gln Glu Val Leu Ser
       130                 135                 140
   Ala Ser Arg Leu Glu Lys Phe Lys His Val Arg Phe Ala Arg Ile Gln
   145                 150                 155                 160
   Ala Ser Ile Lys Asn Leu Tyr Thr Arg Ile Asp Gly Asn Ser Ser Thr
                   165                 170                 175
   Ile Asn Leu Thr Asp Trp Leu Glu Glu Leu Asn Phe Gly Leu Ile Val
               180                 185                 190
   Lys Met Ile Ala Gly Lys Asn Tyr Glu Ser Gly Lys Gly Asp Glu Gln
           195                 200                 205
   Val Glu Arg Phe Lys Lys Ala Phe Lys Asp Phe Met Ile Leu Ser Met
       210                 215                 220
   Glu Phe Val Leu Trp Asp Ala Phe Pro Ile Pro Leu Phe Lys Trp Val
   225                 230                 235                 240
   Asp Phe Gln Gly His Val Lys Ala Met Lys Arg Thr Phe Lys Asp Ile
                   245                 250                 255
   Asp Ser Val Phe Gln Asn Trp Leu Glu Glu His Ile Asn Lys Arg Glu
               260                 265                 270
   Lys Met Glu Val Asn Ala Glu Gly Asn Glu Gln Asp Phe Ile Asp Val
           275                 280                 285
   Val Leu Ser Lys Met Ser Asn Glu Tyr Leu Gly Glu Gly Tyr Ser Arg
       290                 295                 300
   Asp Thr Val Ile Lys Ala Thr Val Phe Ser Leu Val Leu Asp Ala Ala
   305                 310                 315                 320
   Asp Thr Val Ala Leu His Ile Asn Trp Gly Met Ala Leu Leu Ile Asn
                   325                 330                 335
   Asn Gln Lys Ala Leu Thr Lys Ala Gln Glu Glu Ile Asp Thr Lys Val
               340                 345                 350
   Gly Lys Asp Arg Trp Val Glu Glu Ser Asp Ile Lys Asp Leu Val Tyr
           355                 360                 365
   Leu Gln Ala Ile Val Lys Glu Val Leu Arg Leu Tyr Pro Pro Gly Pro
       370                 375                 380
   Leu Leu Val Pro His Glu Asn Val Glu Asp Cys Val Val Ser Gly Tyr
   385                 390                 395                 400
   His Ile Pro Lys Gly Thr Arg Leu Phe Ala Asn Val Met Lys Leu Leu
                   405                 410                 415
   Arg Asp Pro Lys Leu Trp Pro Asp Pro Asp Thr Phe Asp Pro Glu Arg
               420                 425                 430
   Phe Ile Ala Thr Asp Ile Asp Phe Arg Gly Gln Tyr Tyr Lys Tyr Ile
           435                 440                 445
   Pro Phe Gly Ser Gly Arg Arg Ser Cys Pro Gly Met Thr Tyr Ala Leu
       450                 455                 460
   Gln Val Glu His Leu Thr Met Ala His Leu Ile Gln Gly Phe Asn Tyr
   465                 470                 475                 480
   Arg Thr Pro Asn Asp Glu Pro Leu Asp Met Lys Glu Gly Ala Gly Ile
                   485                 490                 495
   Thr Ile Arg Lys Val Asn Pro Val Glu Leu Ile Ile Ala Pro Arg Leu
               500                 505                 510
   Ala Pro Glu Leu Tyr
           515
<210> 5
   <211> 316
   <212> DNA
   <213> Artificial Sequence
<220>
   <223> synthetically generated fragment
<400> 5
   ctgcaacgtg atcctaaact ctggtctgat cctgatactt tcgatccaga gagattcatt 60
   gctactgata ttgactttcg tggtcagtac tataagtata tcccgtttgg ttctggaaga 120
   cgatcttgtc cagggatgac ttatgcattg caagtggaac acttaacaat ggcacatttg 180
   atccaaggtt tcaattacag aactccaaat gacgagccct tggatatgaa ggaaggtgca 240
   ggcataacta tacgtaaggt aaatcctgtg gaactgataa tagcgcctcg cctggcacct 300
   gagctttatt aaaacc                                                 316
<210> 6
   <211> 179
   <212> DNA
   <213> Artificial Sequence
<220>
   <223> synthetically generated fragment
<400> 6
   ggtctgatcg tgaagatgat cgctggaaaa aattatgaat ccggtaaagg agatgaacaa 60
   gtggagagat ttaagaaagc gtttaaggat tttatgattt tatcaatgga gtttgtgtta 120
   tgggatgcat ttccaattcc attatttaaa tgggtggatt ttcaagggca tgttaaggc  179
<210> 7
   <211> 254
   <212> DNA
   <213> Artificial Sequence
<220>
   <223> synthetically generated fragment
<400> 7
   gggatgcatt tccaattcca ttatttaaat gggtggattt tcaagggcat gttaaggcta 60
   tgaaaaggac ttttaaagat atagattctg tttttcagaa ttggttagag gaacatatta 120
   ataaaagaga aaaaatggag gttaatgcag aagggaatga acaagatttc attgatgtgg 180
   tgctttcaaa aatgagtaat gaatatcttg gtgaaggtta ctctcgtgat actgtcatta 240
   aagcaacggt gttt                                                   254
<210> 8
   <211> 120
   <212> DNA
   <213> Artificial Sequence
<220>
<223> synthetically generated fragment
<400> 8
   ttggatatga aggaaggtgc aggcataact atacgtaagg taaatcctgt ggaactgata 60
   atagcgcctc gcctggcacc tgagctttat taaaacctaa gatctttcat cttggttgat 120
<210> 9
   <211> 998
   <212> DNA
   <213> Artificial Sequence
<220>
   <223> synthetically generated fragment
<400> 9
   gtaagttcat ctgtcatttt tcatttattc acttttattt tgaggagcag acatgttaat 60
   aataatttgg agcaactgta aagttatcta tgtgtacagg ttcgagcctc aggtgcaacc 120
   actaatgctt gtattagatt atgttgtctg catcataccc ctaattggag tgtggctctt 180
   cccgaaccct gcaatgctgg atgctggatg ctttatgtat cagactgacc tttttgttaa 240
   actatctaaa tactaaggat gatttaataa aaatatagaa tggtaaacag aaaaagatga 300
   gattattttt ggggctatat ggattcgccc gggctttggg aggtaaaacg gtatctacca 360
   gttgagactt tactccagaa ctttatctcg agagctctga ataaaaatga aatagtattt 420
   accactccaa aatctttgat ggtaaaaaga tgagatataa cctcttataa ttgattgaac 480
   cacgttgata gaataaaact tctttactcc cattcagcat aagaaaaatg aaaccaaacg 540
   gaattcttct cttttttagg gggaaattcc ttaattgctt gttgaatata gattcatgtc 600
   gttattctat ttttaataat gatgaaaatc aatatagtca aagttaatac ttatgtcatt 660
   tggtttgcgg acaagttata ttggaactat ataatacgtc tattatagaa tagtgattat 720
   ttagaggata tacatttttt ttggataaat atttgattta ttggattaaa aatagaatat 780
   acaggtaagg tctaaaacgt gtgtttgctt ttacactaaa taaacttgac ctcgtacaat 840
   tctaagaaaa tatttgaaat aaatgaatta ttttattgtt aatcaattaa aaaaatcata 900
   gtatagatga gatgtgtgca tacttgacaa taactatact aactaaaaca aggtatgtga 960
   ataattgata ttcctttttt aattattctt ttttccag 998
<210> 10
   <211> 431
   <212> DNA
   <213> Artificial Sequence
<220>
   <223> synthetically generated fragment
<400> 10
   gtctgccagt tcagttcgtt gttcacacaa acggtgatac gtacactttt cccggcaata 60
   acatacggcg tgacatcggc ttcaaatggc gtatagccgc cctgatgctc catcacttcc 120
   tgattattga cccacacttt gccgtaatga gtgaccgcat cgaaacgcag cacgatacgc 180
   tggcctgccc aacctttcgg tataaagact tcgcgctgat accagacgtt gcccgcataa 240
   ttacgaatat ctgcatcggc gaactgatcg ttaaaactgc ctggcacagc aattgcccgg 300
   ctttcttgta acgcgctttc ccaccaacgc tgatcaattc cacagttttc gcgatccaga 360
   ctgaatgccc acaggccgtc gagttttttg atttcacggg ttggggtttc tacaggacgt 420
   aaactagtca g 431
<210> 11
   <211> 155
   <212> DNA
   <213> Artificial Sequence
<220>
   <223> synthetically generated fragment
<400> 11